# EUROPEAN PATENT SPECIFICATION

(11) **EP 1 842 558 B1**
(45) Date of publication and mention of the grant of the patent: **13.06.2012**
(21) Application number: 05805115.2
(22) Date of filing: 24.10.2005
(51) Int. Cl.: C12N 15/113, A61K 31/713, A61K 9/127, A61K 47/44

(54) **COMPOSITION FOR INHIBITING EXPRESSION OF TARGET GENE**
ZUSAMMENSETZUNG ZUR HEMMUNG DER EXPRESSION EINES ZIELGENS
COMPOSITION INHIBANT L'EXPRESSION D'UN GENE CIBLE

(30) Priority: 28.01.2005 JP 2005022253
(43) Date of publication of application: 10.10.2007
(73) Proprietor: Kyowa Hakko Kirin Co., Ltd., Tokyo 100-8185 (JP)
(72) Inventor: YAMAUCHI, Masahiro, Sunto-gun, Shizuoka 411-8731 (JP); TOTTORI, Tsuneaki, Suita-shi, Osaka 565-2851 (JP); ISHIHARA, Atsushi, Sunto-gun, Shizuoka 411-8731 (JP); YAGI, Nobuhiro, Sunto-gun, Shizuoka 411-8731 (JP); KATO, Yasuki, Susono-shi, Shizuoka 410-1115i (JP)
(74) Representative: Kalhammer, Georg
(86) International application number: PCT/JP2005/019526
(87) International publication number: WO 2006/080118

(56) References cited:
- WO-A1-02/28367
- WO-A1-2004/105774
- WO-A1-2005/010185
- NANDAN MANDAYAM O ET AL: "Kruppel-like factor 5 mediates the transforming activity of oncogenic H-Ras" ONCOGENE, NATURE PUBLISHING GROUP, GB, vol. 23, no. 19, 22 April 2004 (2004-04-22), pages 3404-3413, XP002340051 ISSN: 0950-9232
- SORENSEN D.R. ET AL: 'Gene Silencing by Systemic Delivery of Synthetic siRNAs in Adult Mice' J.MOL.BIOL. vol. 327, 2003, pages 761 - 766, XP004454177
- SPAGNOU S. ET AL: 'Lipidic Carriers of siRNA: Differences in the Formulation, Cellular Uptake, and Delivery with Plasmid DNA' BIOCHEMISTRY vol. 43, 2004, pages 13348 - 13356, XP008044038
- DALBY B. ET AL: 'Advanced transfection with Lipofectamine 2000 reagent: primary neurons, siRNA, and high-throughput applications' METHODS vol. 33, 2004, pages 95 - 103, XP004505450
- SHINDO T. ET AL: 'Kruppel-like zinc-finger transcription factor KLF5/BTEB2 is a target for angiotensin II signaling and an essential regulator of cardiovascular remodeling' NATURE MEDICINE vol. 8, no. 8, 2002, pages 856 - 863, XP002970412
- MICHAEL D. ET AL: 'A gene encoding an intestinal-enriched member of the Kruppel-like factor family expressed in intestinal epithelial cells' NUCLEIC ACIDS RESEARCH vol. 27, no. 5, 1999, pages 1263 - 1270, XP002904102
- PAUL C.P. ET AL: 'Effective expression of small interfering RNA in human cells' NATURE BIOTECHNOLOGY vol. 20, 2002, pages 505 - 508, XP001121066
- JIANG M. ET AL: 'Gel-Based Application of siRNA to Human Epithelial Cancer Cells Induces RNAi-Dependent Apoptosis' OLIGONUCLEOTIDES vol. 14, 2004, pages 239 - 248, XP002321807

### [SEQUENCE LISTING]

### SEQUENCE LISTING

<110> Kyowa Hakko Kogyo Co., Ltd.
<120> Preparation which inhibit gene expression
<130> 1743
<160> 42
<170> PatentIn version 3.1
<210> 1
   <211> 19
   <212> RNA
   <213> Mus musculus
<220>
   <223> Inventor: Yamauchi, Masahiro; Tottori, Tsuneaki; Ishihara, Atsushi; Yagi, Nobuhiro; Kato, Yasuki
<400> 1
   caugaacguc uuccucccu 19
<210> 2
   <211> 19
   <212> RNA
   <213> Mus musculus
<400> 2
   auuuaccugc cacucugcc 19
<210> 3
   <211> 19
   <212> RNA
   <213> Mus musculus
<400> 3
   ggaguaaccc ggaucugga 19
<210> 4
   <211> 19
   <212> RNA
   <213> Mus musculus
<400> 4
   aagcucaccu gaggacuca 19
<210> 5
   <211> 19
   <212> RNA
   <213> Mus musculus
<400> 5
   uccccagacc guccaugcc 19
<210> 6
   <211> 19
   <212> RNA
   <213> Mus musculus
<400> 6
   cgcugcgccc acccgccug 19
<210> 7
   <211> 19
   <212> RNA
   <213> Mus musculus
<400> 7
   auggagaagu aucugaccc 19
<210> 8
   <211> 19
   <212> RNA
   <213> Mus musculus
<400> 8
   aguauagacg agacagugc 19
<210> 9
   <211> 19
   <212> RNA
   <213> Mus musculus
<400> 9
   accagacggc aguaaugga 19
<210> 10
   <211> 19
   <212> RNA
   <213> Mus musculus
<400> 10
   gcucagagcc uggaagucc 19
<210> 11
   <211> 19
   <212> RNA
   <213> Mus musculus
<400> 11
   gccguuccag ugcauggug 19
<210> 12
   <211> 19
   <212> RNA
   <213> Homo sapiens
<400> 12
   auuuacccac cacccugcc 19
<210> 13
   <211> 19
   <212> RNA
   <213> Homo sapiens
<400> 13
   ggaguaaccc cgauuugga 19
<210> 14
   <211> 19
   <212> RNA
   <213> Homo sapiens
<400> 14
   auggagaagu aucugacac 19
<210> 15
   <211> 19
   <212> RNA
   <213> Homo sapiens
<400> 15
   aucagacagc agcaaugga 19
<210> 16
   <211> 19
   <212> RNA
   <213> Homo sapiens
<400> 16
   gcccuuccag ugcggggug 19
<210> 17
   <211> 21
   <212> RNA
   <213> Artificial
<220>
   <223> siRNA No. 1 sense strand
<220>
   <221> misc_feature
   <222> (20) .. (21)
   <223> DNA
<400> 17
   caugaacguc uuccucccut t 21
<210> 18
   <211> 21
   <212> RNA
   <213> Artificial
<220>
   <223> siRNA No. 1 antisense strand
<220>
   <221> misc_feature
   <222> (20) .. (21)
   <223> DNA
<400> 18
   agggaggaag acguucaugt t 21
<210> 19
   <211> 21
   <212> RNA
   <213> Artificial
<220>
   <223> siRNA No. 2 sense strand
<400> 19
   auuuaccugc cacucugccu u 21
<210> 20
   <211> 21
   <212> RNA
   <213> Artificial
<220>
   <223> siRNA No. 2 antisense strand
<400> 20
   ggcagagugg cagguaaauu u 21
<210> 21
   <211> 21
   <212> RNA
   <213> Artificial
<220>
   <223> siRNA No. 3 sense strand
<400> 21
   ggaguaaccc ggaucuggau u 21
<210> 22
   <211> 21
   <212> RNA
   <213> Artificial
<220>
   <223> siRNA No. 3 antisense strand
<400> 22
   uccagauccg gguuacuccu u 21
<210> 23
   <211> 21
   <212> RNA
   <213> Artificial
<220>
   <223> siRNA No. 4 sense strand
<400> 23
   aagcucaccu gaggacucau u 21
<210> 24
   <211> 21
   <212> RNA
   <213> Artificial
<220>
   <223> siRNA No. 4 antisense strand
<400> 24
   ugaguccuca ggugagcuuu u 21
<210> 25
   <211> 21
   <212> RNA
   <213> Artificial
<220>
   <223> siRNA No. 5 sense strand
<400> 25
   uccccagacc guccaugccu u 21
<210> 26
   <211> 21
   <212> RNA
   <213> Artificial
<220>
   <223> siRNA No. 5 antisense strand
<400> 26
   ggcauggacg gucugggggu u 21
<210> 27
   <211> 21
   <212> RNA
   <213> Artificial
<220>
   <223> siRNA No. 6 sense strand
<400> 27
   cgcugcgccc acccgccugu u 21
<210> 28
   <211> 21
   <212> RNA
   <213> Artificial
<220>
   <223> siRNA No. 6 antisense strand
<400> 28
   caggcgggug ggcgcagcgu u 21
<210> 29
   <211> 21
   <212> RNA
   <213> Artificial
<220>
   <223> siRNA No. 7 sense strand
<400> 29
   auggagaagu aucugacccu u 21
<210> 30
   <211> 21
   <212> RNA
   <213> Artificial
<220>
   <223> siRNA No. 7 antisense strand
<400> 30
   gggucagaua cuucuccauu u 21
<210> 31
   <211> 21
   <212> RNA
   <213> Artificial
<220>
   <223> siRNA No. 8 sense strand
<400> 31
   aguauagacg agacagugcu u 21
<210> 32
   <211> 21
   <212> RNA
   <213> Artificial
<220>
   <223> siRNA No. 8 antisense strand
<400> 32
   gcacugucuc gucuauacuu u 21
<210> 33
   <211> 21
   <212> RNA
   <213> Artificial
<220>
   <223> siRNA No. 9 sense strand
<400> 33
   accagacggc aguaauggau u 21
<210> 34
   <211> 21
   <212> RNA
   <213> Artificial
<220>
   <223> siRNA No. 9 antisense strand
<400> 34
   uccauuacug ccgucuggcu u 21
<210> 35
   <211> 21
   <212> RNA
   <213> Artificial
<220>
   <223> siRNA No. 10 sense strand
<400> 35
   gcucagagcc uggaaguccu u 21
<210> 36
   <211> 21
   <212> RNA
   <213> Artificial
<220>
   <223> siRNA No. 10 antisense strand
<400> 36
   ggacuuccag gcucugagcu u 21
<210> 37
   <211> 21
   <212> RNA
   <213> Artificial
<220>
   <223> siRNA No. 11 sense strand
<400> 37
   gccguuccag ugcauggugu u 21
<210> 38
   <211> 21
   <212> RNA
   <213> Artificial
<220>
   <223> siRNA No. 11 antisense strand
<400> 38
   caccaugcac uggaacggcu u 21
<210> 39
   <211> 21
   <212> RNA
   <213> Artificial
<220>
   <223> SEAP-siRNA sense strand
<400> 39
   agggcaacuu ccagaccauu u 21
<210> 40
   <211> 21
   <212> RNA
   <213> Artificial
<220>
   <223> SEAP-siRNA antisense strand
<400> 40
   auggucugga aguugcccuu u 21
<210> 41
   <211> 1591
   <212> DNA
   <213> Mus musculus
<220>
   <221> CDS
   <222> (167)..(1507)
   <223>
<400> 41
   ccgagcccag gagccccgat ctccgtgccc gccttcgtga gcgtctggct gccggcccag      60
   gggtcccccg ccgcggcccc ccgccgagtc cgccgtcccg tgccagcccg agcgaggtgg     120
   gatcgcgatc gctccgtgtc ccgctcccgt aatccccaga ccgtcc atg ccc acg       175
                                                       Met Pro Thr
                                                       1
   cgg gtg ctg acc atg agc gcc cgc ctg gga cca ctg ccc cag ccg ccg      223
   Arg Val Leu Thr Met Ser Ala Arg Leu Gly Pro Leu Pro Gln Pro Pro
       5                   10                  15
   gcc gcg cag gcc gag ccc gtg ttc gcg cag ctc aag ccg gtg ctg ggc      271
   Ala Ala Gln Ala Glu Pro Val Phe Ala Gln Leu Lys Pro Val Leu Gly
   20                  25                  30                  35
   gct gcg aac ccg gcc cgc gac gcg gcg ctc ttc tcc gga gac gat ctg      319
   Ala Ala Asn Pro Ala Arg Asp Ala Ala Leu Phe Ser Gly Asp Asp Leu
                   40                  45                  50
   aaa cac gcg cac cac cac ccg cct gcg ccg ccg cca gcc gct ggc ccg      367
   Lys His Ala His His His Pro Pro Ala Pro Pro Pro Ala Ala Gly Pro
               55                  60                  65
   cga ctg ccc tcg gag gag ctg gtc cag aca aga tgt gaa atg gag aag      415
   Arg Leu Pro Ser Glu Glu Leu Val Gln Thr Arg Cys Glu Met Glu Lys
           70                  75                  80
   tat ctg acc cct cag ctc cct cca gtt ccg ata att tca gag cat aaa      463
   Tyr Leu Thr Pro Gln Leu Pro Pro Val Pro Ile Ile Ser Glu His Lys
       85                  90                  95
   aag tat aga cga gac agt gcc tca gtg gta gac cag ttc ttc act gac      511
   Lys Tyr Arg Arg Asp Ser Ala Ser Val Val Asp Gln Phe Phe Thr Asp
   100                 105                 110                 115
   act gaa ggc ata cct tac agc atc aac atg aac gtc ttc ctc cct gac      559
   Thr Glu Gly Ile Pro Tyr Ser Ile Asn Met Asn Val Phe Leu Pro Asp
                   120                 125                 130
   atc act cac ctg aga act ggc ctc tac aaa tcc cag aga cca tgc gta      607
   Ile Thr His Leu Arg Thr Gly Leu Tyr Lys Ser Gln Arg Pro Cys Val
               135                 140                 145
   aca cag atc aag aca gaa cct gtt acc att ttc agc cac cag agc gag      655
   Thr Gln Ile Lys Thr Glu Pro Val Thr Ile Phe Ser His Gln Ser Glu
           150                 155                 160
   tcg acg gcc cct cct cct cct ccg gcc ccc acc cag gct ctc ccc gag      703
   Ser Thr Ala Pro Pro Pro Pro Pro Ala Pro Thr Gln Ala Leu Pro Glu
       165                 170                 175
   ttc act agt atc ttc agc tcc cac cag acc aca gcg cca cca cag gag      751
   Phe Thr Ser Ile Phe Ser Ser His Gln Thr Thr Ala Pro Pro Gln Glu
   180                 185                 190                 195
   gtg aac aat atc ttc atc aaa caa gaa ctt cct ata cca gat ctt cat      799
   Val Asn Asn Ile Phe Ile Lys Gln Glu Leu Pro Ile Pro Asp Leu His
                   200                 205                 210
   ctc tct gtc cct tcc cag cag ggc cac ctg tac cag ctg ttg aat aca      847
   Leu Ser Val Pro Ser Gln Gln Gly His Leu Tyr Gln Leu Leu Asn Thr
               215                 220                 225
   ccg gat cta gac atg ccc agt tcg aca aac cag acg gca gta atg gac      895
   Pro Asp Leu Asp Met Pro Ser Ser Thr Asn Gln Thr Ala Val Met Asp
           230                 235                 240
   acc ctt aat gtt tct atg gca ggc ctt aac cca cac ccc tct gct gtt      943
   Thr Leu Asn Val Ser Met Ala Gly Leu Asn Pro His Pro Ser Ala Val
       245                 250                 255
   cca cag acg tca atg aaa cag ttc cag ggc atg ccc cct tgc acg tac      991
   Pro Gln Thr Ser Met Lys Gln Phe Gln Gly Met Pro Pro Cys Thr Tyr
   260                 265                 270                 275
   acc atg cca agt cag ttt ctt cca cag cag gcc act tat ttt ccc ccg     1039
   Thr Met Pro Ser Gln Phe Leu Pro Gln Gln Ala Thr Tyr Phe Pro Pro
                   280                 285                 290
   tca cca cca agc tca gag cct gga agt ccc gat aga caa gct gag atg      1087
   Ser Pro Pro Ser Ser Glu Pro Gly Ser Pro Asp Arg Gln Ala Glu Met
               295                 300                 305
   ctg cag aat ctc acc cca cct ccg tcc tat gcc gct aca att gct tcc      1135
   Leu Gln Asn Leu Thr Pro Pro Pro Ser Tyr Ala Ala Thr Ile Ala Ser
           310                 315                 320
   aaa ctg gcg att cac aac cca aat tta cct gcc act ctg cca gtt aat      1183
   Lys Leu Ala Ile His Asn Pro Asn Leu Pro Ala Thr Leu Pro Val Asn
       325                 330                 335
   tcg cca act ctc cca cct gtc aga tac aac aga agg agt aac ccg gat      1231
   Ser Pro Thr Leu Pro Pro Val Arg Tyr Asn Arg Arg Ser Asn Pro Asp
   340                 345                 350                 355
   ctg gag aag cga cgt atc cac ttc tgc gat tat aat ggt tgc aca aaa      1279
   Leu Glu Lys Arg Arg Ile His Phe Cys Asp Tyr Asn Gly Cys Thr Lys
                   360                 365                 370
   gtt tat aca aag tcg tct cac tta aaa gct cac ctg agg act cat acg      1327
   Val Tyr Thr Lys Ser Ser His Leu Lys Ala His Leu Arg Thr His Thr
               375                 380                 385
   ggc gag aag ccc tac aag tgc acc tgg gag ggc tgc gac tgg agg ttt      1375
   Gly Glu Lys Pro Tyr Lys Cys Thr Trp Glu Gly Cys Asp Trp Arg Phe
           390                 395                 400
   gcc cgg tcg gat gag ctg acc cgc cac tac agg aag cac acg ggc gcc      1423
   Ala Arg Ser Asp Glu Leu Thr Arg His Tyr Arg Lys His Thr Gly Ala
       405                 410                 415
   aag ccg ttc cag tgc atg gtg tgc caa cgc agc ttc tcc cgc tcc gac      1471
   Lys Pro Phe Gln Cys Met Val Cys Gln Arg Ser Phe Ser Arg Ser Asp
   420                 425                 430                 435
   cac ctc gcg ctg cac atg aag cgc cac cag aac tga gcgagcgaac           1517
   His Leu Ala Leu His Met Lys Arg His Gln Asn
                   440                 445
   gctgcgccca cccgcctgac gccttgcagt ccgctttgcc atcctttaaa ccgcagacct    1577
   aacttcataa aaag                                                      1591
<210> 42
   <211> 3359
   <212> DNA
   <213> Homo sapiens
<220>
   <221> CDS
   <222> (312)..(1685)
<400> 42
   ggtacgtgcg ctcgcggttc tctcgcggag gtcggcggtg gcgggagcgg gctccggaga 60
   gcctgagagc acggtggggc ggggcgggag aaagtggccg cccggaggac gttggcgttt 120
   acgtgtggaa gagcggaaga gttttgcttt tcgtgcgcgc cttcgaaaac tgcctgccgc 180
   tgtctgagga gtccacccga aacctcccct cctccgccgg cagccccgcg ctgagctcgc    240
   cgacccaagc cagcgtgggc gaggtgggaa gtgcgcccga cccgcgcctg gagctgcgcc    300
   cccgagtgcc c atg gct aca agg gtg ctg agc atg agc gcc cgc ctg gga     350
                Met Ala Thr Arg Val Leu Ser Met Ser Ala Arg Leu Gly
                1               5                   10
   ccc gtg ccc cag ccg ccg gcg ccg cag gac gag ccg gtg ttc gcg cag      398
   Pro Val Pro Gln Pro Pro Ala Pro Gln Asp Glu Pro Val Phe Ala Gln
       15                  20                  25
   ctc aag ccg gtg ctg ggc gcc gcg aat ccg gcc cgc gac gcg gcg ctc      446
   Leu Lys Pro Val Leu Gly Ala Ala Asn Pro Ala Arg Asp Ala Ala Leu
   30                  35                  40                  45
   ttc ccc ggc gag gag ctg aag cac gcg cac cac cgc ccg cag gcg cag      494
   Phe Pro Gly Glu Glu Leu Lys His Ala His His Arg Pro Gln Ala Gln
                   50                  55                  60
   ccc gcg ccc gcg cag gcc ccg cag ccg gcc cag ccg ccc gcc acc ggc      542
   Pro Ala Pro Ala Gln Ala Pro Gln Pro Ala Gln Pro Pro Ala Thr Gly
               65                  70                  75
   ccg cgg ctg cct cca gag gac ctg gtc cag aca aga tgt gaa atg gag      590
   Pro Arg Leu Pro Pro Glu Asp Leu Val Gln Thr Arg Cys Glu Met Glu
           80                  85                  90
   aag tat ctg aca cct cag ctt cct cca gtt cct ata att cca gag cat      638
   Lys Tyr Leu Thr Pro Gln Leu Pro Pro Val Pro Ile Ile Pro Glu His
       95                  100                 105
   aaa aag tat aga cga gac agt gcc tca gtc gta gac cag ttc ttc act      686
   Lys Lys Tyr Arg Arg Asp Ser Ala Ser Val Val Asp Gln Phe Phe Thr
   110                 115                 120                 125
   gac act gaa ggg tta cct tac agt atc aac atg aac gtc ttc ctc cct      734
   Asp Thr Glu Gly Leu Pro Tyr Ser Ile Asn Met Asn Val Phe Leu Pro
                   130                 135                 140
   gac atc act cac ctg aga act ggc ctc tac aaa tcc cag aga ccg tgc      782
   Asp Ile Thr His Leu Arg Thr Gly Leu Tyr Lys Ser Gln Arg Pro Cys
               145                 150                 155
   gta aca cac atc aag aca gaa cct gtt gcc att ttc agc cac cag agt      830
   Val Thr His Ile Lys Thr Glu Pro Val Ala Ile Phe Ser His Gln Ser
           160                 165                 170
   gaa acg act gcc cct cct ccg gcc ccg acc cag gcc ctc cct gag ttc      878
   Glu Thr Thr Ala Pro Pro Pro Ala Pro Thr Gln Ala Leu Pro Glu Phe
       175                 180                 185
   acc agt ata ttc agc tca cac cag acc gca gct cca gag gtg aac aat      926
   Thr Ser Ile Phe Ser Ser His Gln Thr Ala Ala Pro Glu Val Asn Asn
   190                 195                 200                 205
   att ttc atc aaa caa gaa ctt cct aca cca gat ctt cat ctt tct gtc      974
   Ile Phe Ile Lys Gln Glu Leu Pro Thr Pro Asp Leu His Leu Ser Val
                   210                 215                 220
   cct acc cag cag ggc cac ctg tac cag cta ctg aat aca ccg gat cta     1022
   Pro Thr Gln Gln Gly His Leu Tyr Gln Leu Leu Asn Thr Pro Asp Leu
               225                 230                 235
   gat atg ccc agt tct aca aat cag aca gca gca atg gac act ctt aat      1070
   Asp Met Pro Ser Ser Thr Asn Gln Thr Ala Ala Met Asp Thr Leu Asn
           240                 245                 250
   gtt tct atg tca gct gcc atg gca ggc ctt aac aca cac acc tct gct      1118
   Val Ser Met Ser Ala Ala Met Ala Gly Leu Asn Thr His Thr Ser Ala
       255                 260                 265
   gtt ccg cag act gca gtg aaa caa ttc cag ggc atg ccc cct tgc aca      1166
   Val Pro Gln Thr Ala Val Lys Gln Phe Gln Gly Met Pro Pro Cys Thr
   270                 275                 280                 285
   tac aca atg cca agt cag ttt ctt cca caa cag gcc act tac ttt ccc      1214
   Tyr Thr Met Pro Ser Gln Phe Leu Pro Gln Gln Ala Thr Tyr Phe Pro
                   290                 295                 300
   ccg tca cca cca agc tca gag cct gga agt cca gat aga caa gca gag      1262
   Pro Ser Pro Pro Ser Ser Glu Pro Gly Ser Pro Asp Arg Gln Ala Glu
               305                 310                 315
   atg ctc cag aat tta acc cca cct cca tcc tat gct gct aca att gct      1310
   Met Leu Gln Asn Leu Thr Pro Pro Pro Ser Tyr Ala Ala Thr Ile Ala
           320                 325                 330
   tct aaa ctg gca att cac aat cca aat tta ccc acc acc ctg cca gtt      1358
   Ser Lys Leu Ala Ile His Asn Pro Asn Leu Pro Thr Thr Leu Pro Val
       335                 340                 345
   aac tca caa aac atc caa cct gtc aga tac aat aga agg agt aac ccc      1406
   Asn Ser Gln Asn Ile Gln Pro Val Arg Tyr Asn Arg Arg Ser Asn Pro
   350                 355                 360                 365
   gat ttg gag aaa cga cgc atc cac tac tgc gat tac cct ggt tgc aca      1454
   Asp Leu Glu Lys Arg Arg Ile His Tyr Cys Asp Tyr Pro Gly Cys Thr
                   370                 375                 380
   aaa gtt tat acc aag tct tct cat tta aaa gct cac ctg agg act cac      1502
   Lys Val Tyr Thr Lys Ser Ser His Leu Lys Ala His Leu Arg Thr His
               385                 390                 395
   act ggt gaa aag cca tac aag tgt acc tgg gaa ggc tgc gac tgg agg      1550
   Thr Gly Glu Lys Pro Tyr Lys Cys Thr Trp Glu Gly Cys Asp Trp Arg
           400                 405                 410
   ttc gcg cga tcg gat gag ctg acc cgc cac tac cgg aag cac aca ggc      1598
   Phe Ala Arg Ser Asp Glu Leu Thr Arg His Tyr Arg Lys His Thr Gly
       415                 420                 425
   gcc aag ccc ttc cag tgc ggg gtg tgc aac cgc agc ttc tcg cgc tct      1646
   Ala Lys Pro Phe Gln Cys Gly Val Cys Asn Arg Ser Phe Ser Arg Ser
   430                 435                 440                 445
   gac cac ctg gcc ctg cat atg aag agg cac cag aac tga gcactgcccg       1695
   Asp His Leu Ala Leu His Met Lys Arg His Gln Asn
                   450                 455
   tgtgacccgt tccaggtccc ctgggctccc tcaaatgaca gacctaacta ttcctgtgta    1755
   aaaacaacaa aaacaaaaaa aaaacaagaa aaccacaact aaaactggaa atgtatattt   1815
   tgtatatttg agaaaacagg gaatacattg tattaatacc aaagtgtttg gtcattttaa   1875
   gaatctggaa tgcttgctgt aatgtatatg gctttactca agcagatctc atctcatctc   1935
   atgacaggca gccagtctca acatgggtaa ggggtggggg tgaaggggag tgtgtgcagc   1995
   gtttttacct aggcaccatc atttaatgtg acagtgttca gtaaacaaat cagttggcag   2055
   gcaccagaag aagaatggat tgtatgtcaa gattttactt ggcattgagt agtttttttc   2115
   aatagtaggt aattccttag agatacagta tacctggcaa ttcacaaata gccattgaac   2175
   aaatgtgtgg gtttttaaaa attatataca tatatgagtt gcctatattt gctattcaaa   2235
   attttgtaaa tatgcaaatc agctttatag gtttattaca agttttttag gattcttttg    2295
   gggaagagtc ataattcttt tgaaaataac catgaataca cttacagtta ggatttgtgg   2355
   taaggtacct ctcaacatta ccaaaatcat ttctttagag ggaaggaata atcattcaaa   2415
   tgaactttaa aaaagcaaat ttcatgcact gattaaaata ggattatttt aaatacaaaa   2475
   ggcattttat atgaattata aactgaagag cttaaagata gttacaaaat acaaaagttc   2535
   aacctcttac aataagctaa acgcaatgtc atttttaaaa agaaggactt aggggtcgtt   2595
   ttcacatatg acaatgttgc atttatgatg cagttttcaa gtaccaaaac gttgaattga   2655
   tgatgcagtt ttcatatatc gagatgttcg ctcgtgcagt actgttggtt aaatgacaat   2715
   ttatgtggat tttgcatgta atacacagtg agacacagta attttatcta aattacagtg   2775
   cagtttagtt aatctattaa tactgactca gtgtctgcct ttaaatataa atgatatgtt   2835
   gaaaacttaa ggaagcaaat gctacatata tgcaatataa aatagtaatg tgatgctgat   2895
   gctgttaacc aaagggcaga ataaataagc aaaatgccaa aaggggtctt aattgaaatg   2955
   aaaatttaat tttgttttta aaatattgtt tatctttatt tattttgtgg taatatagta   3015
   agttttttta gaagacaatt ttcataactt gataaattat agttttgttt gttagaaaag   3075
   ttgctcttaa aagatgtaaa tagatgacaa acgatgtaaa taattttgta agaggcttca   3135
   aaatgtttat acgtggaaac acacctacat gaaaagcaga aatcggttgc tgttttgctt   3195
   ctttttccct cttatttttg tattgtggtc atttcctatg caaataatgg agcaaacagc   3255
   tgtatagttg tagaattttt tgagagaatg agatgtttat atattaacga caattttttt   3315
   tttggaaaat aaaaagtgcc taaaagaaaa aaaaaaaaaa aaaa                    3359

## Description

### Technical Field

The present invention relates to a composition for suppressing the expression of a target gene, and the like.

### Background Art

As a method of suppressing the expression of a target gene, for example, a method utilizing RNA interference (hereinafter referred to as RNAi) and the like are known, and specifically, a phenomenon in which when a double-stranded RNA having a sequence identical to that of a target gene is introduced into Nematoda, the expression of the target gene is specifically suppressed has been reported (see "Nature", Vol. 391, No. 6669, pp. 806-811, 1998). Further, it has been found that even when a double-stranded RNA having a length of 21 to 23 nucleotides is introduced into Drosophila, instead of a long double-stranded RNA, the expression of a target gene is suppressed. This is named short interfering RNA (siRNA) (see International Publication No. WO 01/75164).

In the case of mammalian cells, when a long double-stranded RNA was introduced, apoptosis took place as a result of the functions of virus defense mechanism, and thus the expression of a specific gene could not be suppressed. However, it has been found that when siRNA having a length of 20 to 29 nucleotides is used, such a reaction does not take place, and that the expression of a specific gene can be suppressed. Among others, siRNA having 21 to 25 nucleotides has a high effect of suppressing expression ("Nature", Vol. 411, No. 6836, pp. 494-498, 2001; "Nature Reviews Genetics", Vol. 3, No. 10, pp. 737-747, 2002; "Molecular Cell", (USA) Vol. 10, No. 3, pp. 549-561, 2002; "Nature Biotechnology", (USA) Vol. 20, No. 5, pp. 497-500, 2002). In addition, it has also been reported that not only a double-stranded RNA, but also a single-stranded RNA having a hairpin structure as a result of intramolecular hybridization, exhibits RNAi, as with siRNA (see "Proceedings of the National Academy of Sciences of the United States of America", Vol. 99, No. 9, pp. 6047-6052, 2002).

RNAi has been frequently verified in also in vivo tests. The effect of RNAi using siRNA with a length of 50 bp or less on fetal animals (see Patent document 1) and the effect thereof on adult mice (see Patent document 2) have been reported. Moreover, when siRNA is intravenously administered to a fetal mouse, the effect of suppressing the expression of a specific gene has been found in various organs such as kidney, spleen, lung, pancreas, and liver (see Non-patent document 1). Furthermore, it has been reported that when siRNA is directly administered to brain cells, the expression of a specific gene is also suppressed (see Non-patent document 2).

In the Patent document 1 and the Non-patent documents 1 and 2, in vivo administration of siRNA is carried out by local administration or systemic administration using a method of infusing a large amount of an siRNA solution all at once, which is called the hydrodynamic method. siRNA is unstable in the blood, and such an administration method is selected in order to avoid the degradation thereof in the blood. However, by the local administration, siRNA can be delivered only to the vicinity of the administered area, therefore, the efficiency of suppression of the expression is generally extremely low. Non-patent document 3 discloses cationic liposome/lipid-based systems formulated from different molar ratios of the cationic cholesterol-based polyamine lipid *N*¹-cholesteryloxycarbonyl-3,7-diazanonane-1,9-diamine and the neutral helper lipid dioleoyl-L-α-phosphatidylethanolamine. The results of the study show that most commercially available cationic liposome/lipid-based systems investigated mediate a significant nonspecific downregulation of the total cellular protein content at optimal doses for maximal specific gene silencing and knockdown.
On the other hand, in the hydrodynamic method, the target tissue is generally limited to liver.

That is, for the purpose of suppressing the expression of a target gene, development of a method of efficiently delivering an RNA capable of suppressing the expression of the target gene to a target tissue by systemic administration has been demanded.

On the other hand, as means for delivering a nucleic acid into a cell, a method using cationic liposome or cationic polymers is known. However, by the method, after intravenous administration of cationic liposome or cationic polymers comprising a nucleic acid is carried out, the nucleic acid is promptly removed from the blood, and when a target tissue is different from liver or lung, for example, when it is a tumor site or the like, the nucleic acid cannot be delivered to the target tissue, and therefore, the expression of a sufficient action has not been made possible yet. Accordingly, a nucleic acid-encapsulating liposome (liposome encapsulating a nucleic acid therein) with which the problem that a nucleic acid is promptly removed from the blood was solved has been reported (see Patent documents 3 to 6, and Non-patent document 4). In the Patent document 3, as a method of producing liposome comprising a nucleic acid or the like, for example, a method of producing an ODN-encapsulating liposome by dissolving a cationic lipid in chloroform in advance, adding an aqueous solution of oligodeoxynucleotide (ODN) and methanol thereto and mixing and centrifuging the mixture thereby transferring a complex of the cationic lipid and ODN to a chloroform layer, and then taking out the chloroform layer, adding a polyethylene glycolated phospholipid, a neutral lipid and water to the chloroform layer to form a water-in-oil (w/o) emulsion and treating the emulsion by the reverse phase evaporation method has been reported. In the Patent document 4 and the Non-patent document 4, a method of producing an ODN-encapsulating liposome by dissolving ODN in an aqueous solution of citric acid at pH 3.8, adding a lipid (in ethanol) to the solution, reducing the ethanol concentration to 20 v/v% to prepare an ODN-encapsulating liposome, performing filtration for sizing, removing excess ethanol by dialysis, and then further performing dialysis of the sample at pH 7.5 to remove ODN adhering to the surface of the liposome has been reported. In each method, liposome encapsulating an active ingredient such as a nucleic acid is produced. Patent document 5 discloses a complex of a cationic liposome with an oligo nucleic acid, wherein the liposome comprises 2-O-(2-diethylaminoethyl)carbamoyl-1,3-O-dioleoylglycerol and a phospholipid as main ingredients. The liposome disclosed therein is prepared by mixing the glycerol derivative with the phospholipid and then dispersing the mixture in an aqueous solution, optionally by using an emulsifying dispersion machine.

On the other hand, in the Patent document 6, it has been reported that liposome encapsulating an active ingredient such as a nucleic acid is produced by a method of coating fine particles with a lipid membrane in a liquid. In the method, fine particles are coated with a lipid membrane by reducing the ratio of a polar organic solvent in an aqueous solution comprising the polar organic solvent in which the fine particles are dispersed and a lipid is dissolved. The coating is carried out in the liquid, and for example, fine particles coated with a lipid membrane (coated fine particles) having a size suitable for fine particles for intravenous injection and the like are produced very efficiently. In addition, as an example of the fine particles, for example, a complex which comprises a water-soluble drug and a cationic lipid and formed by an electrostatic interaction is exemplified in the Patent document 6. It has been reported that the particle diameter of coated fine particles obtained by coating the complex particles varies depending on the complex particles to be coated, however, the coated fine particles obtained by coating the ODN-lipid complex have a small particle diameter and can be used as an injection, and the coated complex particles show a high retention in the blood and are much accumulated in a tumor tissue when they are intravenously administered.
Patent document 1: International Publication No. WO 02/132788
Patent document 2: International Publication No. WO 03/10180
Patent document 3: Published Japanese translation of a PCT international application No. 2002-508765
Patent document 4: Published Japanese translation of a PCT international application No. 2002-501511
Patent document 5: International Publication No. WO 2004/105774 A1
Patent document 6: International Publication No. WO 02/28367

Non-patent document 1: "Nature Genetics", Vol. 32, No. 1, pp. 107-108, 2002
Non-patent document 2: "Nature Biotechnology", Vol. 20, No. 10, pp. 1006-1010, 2002
Non-patent document 3: "Biochemistry", Vol. 43, No. 42, pp. 13348-13356, 2004
Non-patent document 4: "Biochimica et Biophysica Acta", Vol. 1510, pp. 152-166, 2001

### Disclosure of the invention

### Problems to be Solved by the Invention

An object of the present invention is to provide a composition for suppressing the expression of a target gene, and the like.

The present invention is defined in the claims. The present disclosure further relates to the following (1) to (37).
(1) A composition comprising an RNA-encapsulated liposome, wherein the RNA comprises a sequence consisting of 15 to 30 contiguous nucleotides of a target gene mRNA and a sequence complementary to the sequence, and the liposome is capable of reaching a tissue or an organ including an expression site of the target gene.
(2) The composition according to the above (1), wherein the liposome is liposome having a size that allows intravenous administration.
(3) The composition according to the above (1) or (2), wherein the RNA is an RNA having an action of suppressing the expression of the target gene utilizing RNA interference (RNAi).
(4) The composition according to any one of above (1) to (3), wherein the target gene is a gene associated with a tumor or an inflammation.
(5) The composition according to any one of above (1) to (4), wherein the target gene is a gene associated with angiogenesis.
(6) The composition according to any one of above (1) to (3), wherein the mRNA is KLF5 mRNA.
(7) The composition according to any one of above (1) to (3), wherein the mRNA is either human or mouse KLF5 mRNA.
(8) The composition according to the above (6) or (7), wherein the RNA is a double-stranded RNA, which has a strand of a sequence consisting of 15 to 30 contiguous nucleotides of KLF5 mRNA and a strand of a sequence complementary to the sequence, except that 1 to 6 nucleotides, which are the same or different, are added to the 3' -terminus of each of the strands in the same or a different manner.
(9) The composition according to the above (6) or (7), wherein the RNA is an RNA, which has a hairpin structure, and in which an RNA having a sequence consisting of 15 to 30 contiguous nucleotides of KLF5 mRNA is ligated to an RNA having a sequence complementary to the sequence via a spacer oligonucleotide, and 1 to 6 nucleotides, which are the same or different, are added to the 3'-terminus thereof.
(10) The composition according to the abode (6) or (7), wherein the RNA is an RNA selected from the group consisting of the following (a) to (c):
   (a) a double-stranded RNA, which has a strand of a sequence shown in any one of SEQ ID numbers: 2 to 16 and a strand of a sequence complementary to the sequence, except that 2 to 4 members, which are the same or different, selected from uridylic acids and deoxythymidylic acids are added to the 3' -terminus of each of the strands in the same or a different manner;
   (b) an RNA, which has a hairpin structure, and in which an RNA having a sequence shown in any one of SEQ ID numbers: 2 to 16 is ligated to an RNA having a sequence complementary to the sequence via a spacer oligonucleotide having 2 uridylic acids or deoxythymidylic acids at the 5'-terminus thereof, and 2 to 4 members, which are the same or different, selected from uridylic acids and deoxythymidylic acids are added to the 3'-terminus thereof; and
   (c) a double-stranded RNA, which has a strand of a sequence shown in any one of SEQ ID numbers: 2 to 11 and a strand of a sequence complementary to the sequence, except that 2 uridylic acids are added to the 3'-terminus of each of the strands.
(11) The composition according to any one of above (1) to (3), wherein the mRNA is bcl2 mRNA.
(12) The composition according to any one of above (1) to (11), wherein the RNA-encapsulated liposome comprises complex particles comprising as constituent components a lead particle and the RNA, and a lipid membrane for coating the complex particles, wherein constituent components of the lipid membrane can be solved in a polar organic solvent, and wherein the polar organic solvent can be contained in a liquid at such a concentration that the constituent components of the lipid membrane are dispersible and the complex particles are dispersible.
(13) The composition according to the above (12), wherein the polar organic solvent is an alcohol.
(14) The composition according to the above (12), wherein the polar organic solvent is ethanol.
(15) The composition according to any one of the above (12) to (14), wherein the lead particle is a lead particle comprising a cationic lipid, and the lipid membrane contains, as constituent components, a neutral lipid and a lipid derivative, a fatty acid derivative or an aliphatic hydrocarbon derivative, of a water-soluble substance.
(16) The composition according to any one of above (1) to (11), wherein the RNA-encapsulated liposome is liposome which comprises complex particles comprising as constituent components a lead particle comprising a cationic lipid and the RNA, and a lipid membrane for coating the complex particles, and the lipid membrane contains, as constituent components, a neutral lipid and a lipid derivative, a fatty acid derivative or an aliphatic hydrocarbon derivative of a water-soluble substance.
(17) The composition according to the above (15) or (16), wherein the cationic lipid is one or more compounds selected from N-[1-(2,3-dioleoylpropyl)]-N,N,N-trimethylammonium chloride, N-[1-(2,3-dioleoylpropyl)]-N,N-dimethylamine, N-[1-(2,3-dioleyloxypropyl)-N,N,N-trimethylammonium chloride, N-[1-(2,3-ditetradecyloxypropyl)]-N,N-dimethyl-N-hydroxyethylammonium bromide and 3β-[N-(N'N'-dimethylaminoethyl)carbamoyl cholesterol.
(18) The composition according to any one of the above (15) to (17), wherein the lipid derivative, the fatty acid derivative or the aliphatic hydrocarbon derivative, of a water-soluble substance is polyethylene glycol phosphatidyl ethanolamine.
(19) The composition according to any one of the above (15) to (18), wherein the neutral lipid is egg yolk phosphatidylcholine.
(20) An RNA-encapsulated liposome comprising complex particles comprising as constituent components a lead particle and an RNA comprising a sequence consisting of 15 to 30 contiguous nucleotides of a target gene mRNA and a sequence complementary to the sequence, and a lipid membrane for coating the complex particles, wherein constituent components of the lipid membrane can be solved in a polar organic solvent, and wherein the polar organic solvent can be contained in a liquid at such a concentration that the constituent components of the lipid membrane are dispersible and the complex particles are dispersible.
(21) The liposome according to the above (20), wherein the polar organic solvent is an alcohol:
(22) The liposome according to the above (20), wherein the polar organic solvent is ethanol.
(23) The liposome according to any one of the above (20) to (22), wherein the lead particle is a lead particle comprising a cationic lipid, and the lipid membrane contains, as constituent components, a neutral lipid and a lipid derivative, a fatty acid derivative or an aliphatic hydrocarbon derivative, of a water-soluble substance.
(24) An RNA-encapsulated liposome comprising complex particles comprising as constituent components a lead particle comprising a cationic lipid and an RNA comprising a sequence consisting of 15 to 30 contiguous nucleotides of a target gene mRNA and a sequence complementary to the sequence, and a lipid membrane for coating the complex particles, wherein the lipid membrane contains, as constituent components, a neutral lipid and a lipid derivative, a fatty acid derivative or an aliphatic hydrocarbon derivative, of a water-soluble substance.
(25) The liposome according to the above (23) or (24), wherein the cationic lipid is one or more compounds selected from N-[1-(2,3-dioleoylpropyl)]-N,N,N-trimethylammonium chloride, N-[1-(2,3-dioleoylpropyl)]-N,N-dimethylamine, N-[1-(2,3-dioleyloxypropyl)-N,N,N-trimethylammonium chloride, N-[1-(2,3-ditetradecyloxypropyl)]-N,N-dimethyl-N-hydroxyethylammonium bromide and 3β-[N-(N'N'-dimethylaminoethyl)carbamoyl cholesterol.
(26) The liposome according to any one of the above (20) to (25), wherein the lipid derivative, the fatty acid derivative or the aliphatic hydrocarbon derivative, of a water-soluble substance is polyethylene glycol phosphatidyl ethanolamine.
(27) The liposome according to any one of the above (20) to (26), wherein the neutral lipid is egg yolk phosphatidylcholine.
(28) The liposome according to any one of the above (20) to (27), wherein the RNA is an RNA having an action of suppressing the expression of the target gene utilizing RNA interference (RNAi).
(29) The liposome according to any one of the above (20) to (28), wherein the target gene is a gene associated with a tumor or an inflammation.
(30) The liposome according to any one of the above (20) to (28), wherein the target gene is a gene associated with angiogenesis.
(31) The liposome according to any one of the above (20) to (28), wherein the mRNA is KLF5 mRNA.
(32) The liposome according to any one of the above (20) to (28), wherein the mRNA is either human or mouse KLF5 mRNA.
(33) The liposome according to the above (31) or (32), wherein the RNA is a double-stranded RNA, which has a strand of a sequence consisting of 15 to 30 contiguous nucleotides of KLF5 mRNA and a strand of a sequence complementary to the sequence, except that 1 to 6 nucleotides, which are the same or different, are added to the 3' -terminus of each of the strands in the same or a different manner.
(34) The liposome according to the above (31) or (32), wherein the RNA is an RNA, which has a hairpin structure, and in which an RNA having a sequence consisting of 15 to 30 contiguous nucleotides of KLF5 mRNA is ligated to an RNA having a sequence complementary to the sequence via a spacer oligonucleotide, and 1 to 6 nucleotides, which are the same or different, are added to the 3'-terminus thereof.
(35) The liposome according to the above (31) or (32), wherein the RNA is an RNA selected from the group consisting of the following (a) to (c):
   (a) a double-stranded RNA, which has a strand of a sequence shown in any one of SEQ ID numbers: 2 to 16 and a strand of a sequence complementary to the sequence, except that 2 to 4 members, which are the same or different, selected from uridylic acids and deoxythymidylic acids are added to the 3' -terminus of each of the strands in the same or a different manner;
   (b) an RNA, which has a hairpin structure, and in which an RNA having a sequence shown in any one of SEQ ID numbers: 2 to 16 is ligated to an RNA having a sequence complementary to the sequence via a spacer oligonucleotide having 2 uridylic acids or deoxythymidylic acids at the 5'-terminus thereof, and 2 to 4 members, which are the same or different, selected from uridylic acids and deoxythymidylic acids are added to the 3'-terminus thereof ; and
   (c) a double-stranded RNA, which has a strand of a sequence shown in any one of SEQ ID numbers: 2 to 11 and a strand of a sequence complementary to the sequence, except that 2 uridylic acids are added to the 3'-terminus of each of the strands.
(36) The liposome according to any one of the above (20) to (28), wherein the mRNA is bcl2 mRNA.
(37) A composition comprising the liposome according to any one of the above (20) to (36).

### Effect of the Invention

By administering the composition of the present invention comprising liposome encapsulating an RNA comprising a sequence consisting of 15 to 30 contiguous nucleotides of a target gene mRNA and a sequence complementary to the sequence to a mammal or the like, the expression of the target gene can be suppressed.

### Brief Description of the Drawings

Fig. 1 shows the growth of tumor transplanted into a mouse with time. The horizontal axis represents time ( days), and the longitudinal axis represents tumor volume (mm³). ● represents a non-treatment group and ○ represents a group administered with a preparation obtained in Example 1.
Fig. 2 shows the tumor growth curve when a preparation comprising 50 µg of siRNA (Example 1: ○ in the drawing, Comparative example 1: ● in the drawing), an siRNA solution comprising 50 µg of siRNA (Comparative example 2: □ in the drawing, Comparative example 3: ■ in the drawing), or a physiological saline solution (Δ in the drawing) was administered to a tumor-bearing mouse through the tail vein.
Fig. 3 shows the results obtained by counting CD31-positive structures observed in a section of tumor tissue. Saline, Sc-saline, sc-WL, KLF5-saline and KLF5-WL show the numbers of CD31-positive structures in tumors of mice to which a physiological saline solution, Comparative example 3, Comparative example 1, Comparative example 2 and Example 1 were administered, respectively.
Fig. 4 shows the results of quantitative PCR analysis of mRNA extracted from tumors. Saline, Sc-saline, sc-WL, KLF5-saline and KLF5-WL represent a physiological saline solution, Comparative example 3, Comparative example 1, Comparative example 2 and Example 1, respectively.
Fig. 5 shows the results of quantitative determination of KLF5 extracted from tumors. Saline, Sc-saline, sc-WL, KLF5-saline and KLF5-WL represent a physiological saline solution, Comparative example 3, Comparative example 1, Comparative example 2 and Example 1, respectively.
Fig. 6 shows the tumor growth curve when siRNA was directly administered in the vicinity of tumor. Δ represents administration of a physiological saline solution, □ represents administration of scramble-siRNA, and ○ represents administration of KLF5-siRNA.
Fig. 7 shows the changes in the growth of DU145 tumor transplanted into a mouse with time. The horizontal axis represents time (days), and the longitudinal axis represents tumor volume (mm³). ● represents a non-treatment group and ○ represents a group administered with a preparation obtained in Example 3.
Fig. 8 shows the changes in the growth of PC-3 tumor transplanted into a mouse with time. The horizontal axis represents time (days), and the longitudinal axis represents tumor volume (mm³). ● represents a non-treatment group and ○, □ and ■ represent groups administered with preparations obtained in Example 3, Comparative example 4 and Comparative example 5 (naked), respectively.

### Best Mode for Carrying Out the Invention

The target gene in the present invention is not particularly limited as long as it is a gene which produces and expresses mRNA in mammals, and examples thereof include Kruppel-like factor (hereinafter abbreviated as KLF) genes, and preferred examples thereof include KLF5 gene. The KLF family is a family of transcriptional factors, which is characterized in that it has a zinc finger motif at the C-terminus thereof, and examples thereof that have been known include KLF1, KLF2, KLF3, KLF4, KLF5, KLF6, KLF7, KLF8, KLF9, KLF10, KLF11, KLF12, KLF13, KLF14, KLF15, KLF 16 and the like. It has been reported that, in mammals, the KLF family plays an important role in differentiation of various types of tissues or cells, such as erythrocytes, vascular endothelial cells, smooth muscle, skin, and lymphocytes, and also in formation of the pathologic conditions of various types of diseases such as cancer, cardiovascular diseases, cirrhosis, renal diseases, and immune-mediated diseases (The Journal of Biological Chemistry, Vol. 276, No. 37, pp. 34355-34358, 2001; Genome Biology, Vol. 4, No. 2, p. 206, 2003).

Among the KLF family members, KLF5 is also referred to as BTEB2 (basic transcriptional element binding protein 2) or IKLF (intestinal-enriched Kruppel-like factor). The expression of KLF5 in vascular smooth muscle is controlled at the development stage thereof. KLF5 is highly expressed in the vascular smooth muscle of a fetus; whereas its expression is not found in the vascular smooth muscle of a healthy adult. In addition, in the case of the smooth muscle of intima of a blood vessel regenerated after denudation by a balloon catheter, KLF5 is highly expressed. Also, in the smooth muscle in lesions due to arteriosclerosis or restenosis, KLF5 is expressed (Circulation, Vol. 102, No. 20, pp. 2528-2534, 2000). Further, examples of the target gene include B-CELL CLL/LYMPHOMA (hereinafter abbreviated as bcl) genes, and preferred examples thereof include bcl2 gene.

Examples of the RNA in the present invention include an RNA comprising a sequence consisting of 15 to 30, preferably 17 to 25, and more preferably 19 to 23 contiguous nucleotides of the above-mentioned target gene mRNA and a sequence complementary to the sequence, and derivatives in which an oxygen atom or the like contained in a phosphate moiety, an ester moiety or the like in the nucleic acid structure has been substituted with another atom such as a sulfur atom are included. Further, preferred examples of the RNA in the present invention include an RNA having an action of suppressing the expression of the target gene utilizing RNA interference (RNAi). Here, by taking an RNA capable of suppressing the expression of KLF5 gene as an example, the RNA capable of suppressing the expression of the target gene by utilizing RNA interference (RNAi) will be explained. Other genes also have a similar structure and can be obtained by a similar procedure.

The RNA capable of suppressing the expression of
KLF5 gene contains a sequence consisting of 15 to 30, preferably 17 to 25, and more preferably 19 to 23 contiguous nucleotides of KLF5 mRNA (hereinafter referred to as sequence X) and a sequence complementary to the sequence (hereinafter referred to as complementary sequence X'). Examples of such an RNA include: (A) an RNA which is a double-stranded RNA having a strand of sequence X (sense strand) and a strand of complementary sequence X' (antisense strand), in which 1 to 6, and preferably 2 to 4 nucleotides are added to the 3' -terminus of each of the strands in the same or a different manner (hereinafter, an RNA having such a structure is referred to as an siRNA), and which is capable of suppressing the expression of KLF5 gene ; and (B) an RNA, which has a hairpin structure, and in which an RNA having sequence X is ligated to an RNA having complementary sequence X' via a spacer oligonucleotide, and 1 to 6, and preferably 2 to 4 nucleotides are added to the 3'-terminus thereof (hereinafter, such an RNA is referred to as a shRNA) and the like. The bases of the nucleotides to be added to such an RNA may be the same or different, and are independently selected from guanine, adenine, cytosine, thymine and uracil, and either RNA or DNA may be used as the nucleotide, however, either one member or two members selected from uridylic acid (U) and deoxythymidylic acid (dT) is/are preferred. As the spacer oligonucleotide, an RNA consisting of 6 to 12 nucleotides is preferred. As the sequence at the 5'-terminus thereof, two nucleotides, UU, are preferred. Examples of the spacer oligonucleotide include an RNA consisting of the sequence UUCAAGAGA. Either of the two RNA portions that are ligated to each other via the spacer oligonucleotide may be suitable as the RNA on the 5'-terminal side. The sequence X may be any sequence as long as it is a sequence consisting of 15 to 30, preferably 17 to 25, and more preferably 19 to 23 contiguous nucleotides of KLF5 mRNA, however, a sequence consisting of 19 nucleotides designed by the method described in the following (I) is most preferred.

With regard to the RNA having the above structures, the strength of suppression of the expression of KLF5 gene varies depending on the sequence X, and the suppression is sometimes weak. Therefore, a number of sequences are designed as the sequence X (I), RNAs are prepared based on the respective sequences X (II), the RNAs are introduced into cells in which KLF5 gene is expressed and the expression of KLF5 gene is measured (III), and an RNA that suppresses the expression of KLF5 gene more strongly is selected, whereby the RNA of the present disclosure can be obtained.

### (I) Design of sequence X

A sequence portion consisting of 21 nucleotides that begin with AA is extracted from the nucleotide sequence of KLF5 cDNA of an animal in which the gene expression is to be suppressed. The GC content of the extracted sequence is calculated, and several sequences having a GC content between 20% and 80%, preferably between 30% and 70%, and more preferably between 40% and 60%, are selected.

Such a sequence is preferably a sequence in a coding region, which, is located 75 nucleotides or more downstream of a start codon. Information regarding the nucleotide sequence of KLF5 cDNA can be obtained from nucleotide sequence database such as GenBank. For example, with regard to sequence information, the sequence of mouse KLF5 cDNA can be obtained from GenBank Accession No. NM_009769 (SEQ ID) NO: 41), and the sequence of human KLF5 cDNA can be obtained from GenBank Accession No. AF287272 (SEQ ID NO: 42).
AA at the 5'-tarminus is eliminated from the selected sequence, and T is then substituted with U in the sequence. The thus obtained sequence consisting of 19 nucleotides is defined as sequence X.

### (II) Preparation of an RNA

RNA (e.g., siRNA, shRNA) can be prepared as follows based on sequence X selected in (I) above. A case where 2 oligonucleotides in total of either one or two of U or dT are used as oligonucleotides to be added will be described below. However, RNA can also be prepared in the case where other nucleotides are used.

### (i) Case of siRNA

An RNA having a sequence obtained by adding 2 oligonucleotides in total of either one or two of U or dT to the 3'-terminus of sequence X, and an RNA having a sequence obtained by adding 2 oligonucleotides in total of either one or two of U or dT to the 3'-terminus of complementary sequence X', are prepared. Such two RNA portions can be prepared by chemical synthesis or in vitro transcription. Chemical synthesis can be carried out using a DNA synthesizer. Otherwise, it is also possible to ask some manufacturers such as Ambion, Japan Bio services Co., Ltd., or QIAGEN, to carry out such chemical synthesis. The thus chemically synthesized two RNA portions comprising sequences complementary to each other are annealed, so as to prepare a double-stranded RNA consisting of a strand of a sequence X and a strand of a complementary sequence X', in which one or two menbers, which are the same or different, selected from U and dT are added to the 3'-terminus of each of the strands. Annealing can be carried out by heating two RNA portions in a suitable buffer at a temperature between 90°C and 95°C for 1 to 5 minutes, and then cooling them to room temperature over 45 to 60 minutes.

RNA can be prepared via in vitro transcription as follows. First, the following DNA portions are prepared: a DNA having the promoter sequence of T7 RNA polymerase (T7 primer); a DNA having a sequence obtained by substituting U with T in complementary sequence X', adding AA at the 5'-terminus thereof, and further adding to the 3'-terminus thereof a sequence complementary to 8 nucleotides of the 3'-terminus of the T7 primer (DNA1) ; and a DNA having a sequence obtained by substituting U with T in sequence X, adding AA at the 5'-terminus thereof, and further adding to the 3'-terminus thereof a sequence complementary to 8 nucleotides of the 3'-terminus of the T7 primer (DNA2).

The T7 primer and the DNA1 are annealed, and thereafter, they are converted to double-stranded DNA by a DNA polymerase reaction. Using the obtained double-stranded DNA as a template, an in vitro transcription reaction is carried out using T7 RNA polymerase, so as to synthesize an RNA having a sequence obtained by adding UU to the 3'-terminus of sequence X and adding a leader sequence to the 5'-terminus thereof. Likewise, the same reaction as mentioned above is carried out using the T7 primer and the DNA2, so as to synthesize an RNA having a sequence obtained by adding UU to the 3'-terminus of complementary sequence X' and adding a leader sequence to the 5'-terminus thereof.

The two reaction solutions are mixed, and such an in vitro transcription reaction is further continued, so that the two RNA portions having sequences complementary to each other are annealed. Thereafter, the double-stranded DNA used as a template and the leader sequence at the 5'-terminus of each RNA strand are digested with deoxyribonuclease and single-stranded RNA-specific ribonuclease, and then eliminated. The UU portion at the 3'-terminus of each RNA strand remains, without being digested.

The aforementioned reaction can be carried out using a kit such as Silencer · siRNA Construction Kit (manufactured by Ambion). DNA to be annealed with the T7 primer can be chemically synthesized using a DNA synthesizer. Moreover, it is also possible to ask some manufacturers such as Ambion, Japan Bio Services Co., Ltd., Hokkaido System Science Co., Ltd., or QLAGEN, to carry out such chemical synthesis.

### (ii) Case of shRNA

An RNA forming a hairpin structure obtained by ligating an RNA having sequence X to an RNA having complementary sequence X' via a spacer oligonucleotide, and then adding 1 to 6, and preferably 2 to 4 nucleotides. to the 3'-terminus thereof; can be prepared by chemical synthesis using a DNA synthesizer.

### (III) Suppression of the expression of KLF5 gene

The siRNA or shRNA prepared in (II) above is transfected into a cell line that expresses KLF5 gene. As a cell line, the cells of the same animal species as the KLF5 cDNA used as a base of the design of sequence X described in (I) above are used. Examples of a cell line that expresses KLF5 gene may include cell lines derived from smooth muscle, fibroblasts or vascular endothelial cells, such as the fetal mouse fibroblast cell line C3H/10T1/2 (ATCC No. CCL-226) or human umbilical cord vascular endothelial cells. Transfection of the RNA can be carried out using reagents for transfection into animal cells, such as Polyfect Transfection Reagent (manufactured by QIAGEN), TransMessenger Transfection Reagent, Oligofectamine Reagent (manufactured by Invitrogen), or Lipofectamine 2000 (manufactured by Invitrogen). These reagents are mixed with the RNA to form a complex, and the complex is then added to cells.

The expression of KLF5 gene in cells, which were transfected with the RNA can be analyzed by RT-PCR. Total RNA is prepared from cells transfected with the RNA, and from cells which were not transfected with the RNA. Thereafter, cDNA is synthesized from the RNA. Using the synthesized cDNA as a template, PCR is carried out with primers specific to KLF5 gene. The amount of an amplified product derived from KLF5 cDNA is quantified by agarose gel electrophotesis, thereby measuring the expression level of KLF5 gene. An RNA that was transfected into cells in which the expression level of the KLF5 gene is lower than the expression level of the KLF5 gene in cells which were not transfected with the RNA, is selected as an RNA capable of suppressing the expression of KLF5 gene.

An example of the thus selected RNA capable of suppressing the expression of KLF5 gene is a double-stranded RNA consisting of a strand of a sequence shown in any one of SEQ ID numbers: 2 to 11 and a strand of a sequence complementary to the above sequence, in which two uridylic acids are added to the 3'-terminus of each of the strands. This double-stranded RNA consisting of a strand of a sequence shown in any one of SEQ ID numbers: 2 to 11 and a strand of a sequence complementary to the above sequence, in which two uridylic acids are added the 3'-terminus of each of the strands is designed based on the sequence of mouse cDNA, and it suppresses the expression of mouse KLF5 gene. Among such sequences, sequences shown in SEQ ID numbers: 4, 8, and 10, are shared by mouse KLF5 mRNA and human KLF5 mRNA. Thus, a double-stranded RNA consisting of a strand of a sequence shown in any one of SEQ ID numbers: 4, 8, and 10, and a strand of a sequence complementary to the above sequence, in which two uridylic acids are added to the 3'-terminus of each of the strands, is capable of suppressing not only the mouse KLF5 gene but also the human KLF5 gene.

The KLF5 cDNA of a certain animal species "A" used as a base for the design of sequence X described in (I) above is aligned with the KLF5 cDNA of a different animal species "B" based on the sequence homology, so as to obtain sequence Y of the animal species "B" that corresponds to sequence X selected in the animal species "A". When an RNA capable of suppressing the expression of KLF5 gene of the animal species "A" is obtained by the aforementioned method, an RNA obtained by substituting sequence X region and complementary sequence X' region with sequence Y and complementary sequence Y' in the RNA, respectively, is considered to be capable of suppressing KLF5 gene of the animal species "B".

For example, a double-stranded RNA consisting of a strand of a sequence shown in any one of SEQ ID numbers: 2, 3, 7, 9, and 11, based on the sequence of mouse KLF5 cDNA, and a strand of a sequence complementary to the above sequence, in which two uridylic acids are added to the 3'-terminus of each of the strands, is capable of suppressing the expression of mouse KLF5 gene. Accordingly, a double stranded RNA consisting of a strand of a sequence shown in any one of SEQ ID numbers: 12 to 16, which are the corresponding sequences of human KLF5 cDNA, and a strand of a sequence complementary to the above sequence, in which two uridylic acids are added to the 3'-terminus of each of the strands, is considered to be capable of suppressing the expression of the human KLF5 gene.

The liposome in the composition of the present invention (hereinafter referred to as liposome A) is not particularly limited as long as it is liposome which encapsulates an RNA comprising a sequence consisting of 15 to 30 contiguous nucleotides of a target gene mRNA and a sequence complementary to the sequence, and is capable of reaching a tissue or an organ including an expression site of the target gene. Examples thereof include liposome which comprises complex particles comprising as constituent components a lead particle and the RNA, and a lipid membrane for encapsulating the complex particles and the like, and preferred examples thereof include liposome which comprises complex particles comprising as constituent components a lead particle and the RNA, and a lipid membrane for coating the complex particles, wherein constituent components of the lipid membrane can be solved in a polar organic solvent, and wherein the polar organic solvent can be contained in a liquid at such a concentration that the constituent components of the lipid membrane are dispersible and the complex particles are dispersible. Further, examples of the liposome A also include liposome which comprises complex particles comprising as constituent components a lead particle comprising preferably a cationic lipid and the above-mentioned RNA, and a lipid membrane for coating the complex particles, and in which the lipid membrane contains, as constituent components, a neutral lipid and a lipid derivative, a fatty acid derivative or an aliphatic hydrocarbon derivative, of a water-soluble substance, wherein the lipid derivative, fatty acid derivative or aliphatic hydrocarbon derivative of a water-soluble substance is selected from the group consisting of a polyethylene glycolated lipid, a polyethylene glycol sorbitan fatty acid ester, a polyethylene glycol fatty acid ester, a polyglycerolated lipid and a polyglycerol fatty acid ester, and preferred examples thereof include liposome which comprises complex particles comprising as constituent components a lead particle and the RNA, and a lipid membrane for coating the complex particles, and in which the lipid membrane contains, as constituent components, a neutral lipid and a lipid derivative, a fatty acid derivative or an aliphatic hydrocarbon derivative, of a water-soluble substance, wherein the lipid derivative, fatty acid derivative or aliphatic hydrocarbon derivative of a water-soluble substance is selected from the group consisting of a polyethylene glycolated lipid, a polyethylene glycol sorbitan fatty acid ester, a polyethylene glycol fatty acid ester, a polyglycerolated lipid and a polyglycerol fatty acid ester, wherein constituent components of the lipid membrane can be solved in a polar organic solvent, and wherein the polar organic solvent can be contained in a liquid at such a concentration that the constituent components of the lipid membrane are dispersible and the complex particles are dispersible.

The lead particle in the present invention is a fine particle comprising as a constituent component, for example, a lipid assembly, liposome, an emulsion particle, a polymer, a metal colloid, a fine particle preparation or the like. Preferred examples thereof include a fine particle comprising liposome as a constituent component. The lead particle in the present invention may contain, as a constituent component, a complex obtained by combining two or more members selected from a lipid assembly, liposome, an emulsion particle, a polymer, a metal colloid, a fine particle preparation and the like, or may contain, as a constituent component, a complex obtained by combining a lipid assembly, liposome, an emulsion particle, a polymer, a metal colloid, a fine particle preparation or the like with another compound (such as a sugar, a lipid or an inorganic compound).

The lipid assembly or the liposome (hereinafter referred to as liposome B) as the constituent component of the lead particle comprises, for example, a lipid and/or a surfactant or the like. The lipid may be any of a simple lipid, a complex lipid and a derived lipid, and examples thereof include a phospholipid, a glyceroglycolipid, a sphingoglycolipid, a sphingoid, a sterol and the like, and preferred examples thereof include a phospholipid. Further, examples of the lipid also include surfactants (the same definition as the surfactant described below), a polymer (the same definition as the polymer described below, specifically dextran, etc.), and lipid derivative such as a polyoxyethylene derivative (specifically, polyethylene glycol, etc.), and preferred examples include a polyethylene glycolated lipid. Examples of the surfactant include a nonionic surfactant, an anionic surfactant, a cationic surfactant, a zwitterionic surfactant and the like.

Examples of the phospholipid include natural and synthetic phospholipids such as phosphatidylcholine (specifically, soybean phosphatidylcholine, egg yolk phosphatidylcholine (EPC), distearoyl phosphatidylcholine, dipalmitoyl phosphatidylcholine, dimyristoyl phosphatidylcholine, dioleoyl phosphatidylcholine, etc.), phosphatidylethanolamine (specifically, distearoyl phosphatidylethanolamine (DSPE), dipalmitoyl phosphatidylethanolamine, dioleoyl phosphatidylethanolamine, etc.), glycerophospholipid (specifically, phosphatidylserine, phosphatidic acid, phosphatidylglycerol, phosphatidylinositol, lysophosphatidylcholine, etc.) sphingophospholipid (specifically sphingomyelin, ceramide phosphoethanolamine, ceramide phosphoglycerol, ceramide phosphoglycerophosphate, etc.) glycerophosphono lipid, sphingophosphonolipid, natural lecithin (specifically, egg yolk lecithin, soybean lecithin, etc.) and hydrogenated phospholipid (specifically hydrogenated phosphatidylcholine, etc.).

Examples of the glyceroglycolipid include sulfoxyribosyl glyceride, diglycosyl diglyceride, digalactosyl diglyceride, galactosyl diglyceride, glycosyl diglyceride and the like.
Examples of the sphingoglycolipid include galactosyl cerebroside, lactosyl cerebroside, ganglioside and the like.

Examples of the sphingoid include sphingan, icosasphingan, sphingosine, a derivative thereof and the like. Examples of the derivative thereof include those in which -NH₂ of sphingan, icosasphingan, sphingosine or the like is replaced with -NHCO(CH₂)ₓCH₃ (in the formula, x represents an integer of 0 to 18, in particular, 6, 12 or 18 is preferred) and the like.
Examples of the sterol include cholesterol, dihydrocholesterol, lanosterol, β-sitosterol, campesterol, stigmasterol, brassicasterol, ergocasterol, fucosterol, 3β-[N-(N'N'-dimethylaminoethyl)carbamoyl cholesterol (DC-Chol) and the like.

Examples of the lipid different from these include N-[1-(2,3-dioleoylpropyl)]-N,N,N-trimethylammonium chloride (DOTAP), N-[1-(2,3-dioleoylpropyl)]-N,N-dimethylamine (DODAP), N-[1-(2,3-dioleyloxypropyl)- N,N,N-trimethylammonium chloride (DOTMA), 2,3- dioleyloxy-N-[2-(sperminecarboxyamido)ethyl]-N,N-dimethyl-1-propanaminium trifluoroacetate (DOSPA), N-[1-(2,3-ditetradecyloxypropyl)]-N,N-dimethyl-N-hydroxyethylammonium bromide (DMRIE), N-[1-(2,3-dioleyloxypropyl)]-N,N-dimethyl-N- hydroxyethylammonium bromide (DORIE) and the like.

Examples of the nonionic surfactants include polyoxyethylene sorbitan monooleate (specifically, Polysorbate 80, etc.), polyoxyethylene polyoxypropylene glycol (specifically, Pluronic F68, etc.), a sorbitan fatty acid (specifically, sorbitan monolaurate, sorbitan monooleate, etc.), a polyoxyethylene derivative (specifically, polyoxyethylene hydrogenated castor oil 60, polyoxyethylene lauryl alcohol, etc.), a glycerol fatty acid ester and the like.

Examples of the anionic surfactants include acylsarcosine, sodium alkylsulfate, alkylbenzene sulfonate, a sodium fatty acid having 7 to 22 carbon atoms and the like. Specific examples include sodium dodecyl sulfate, sodium lauryl sulfate, sodium cholate, sodium deoxycholate, sodium taurodeoxycholate and the like.

Examples of the cationic surfactants include an alkylamine salt, an acylamine salt, a quaternary ammonium salt, an amine derivative and the like. Specific examples include benzalkonium chloride, an acylaminoethyldiethylamine salt, an N-alkylpolyalkylpolyamine salt, a polyethylene polyamide of fatty acid, cetyltrimethylammonium bromide, dodecyltrimethylammonium bromide, alkylpolyoxyethyleneamine, N-alkylaminopropylamine, a triethanolamine fatty acid ester and the like.

Examples of the zwitterionic surfactants include 3-[3-cholamidopropyl]dimethylammonio]-1-propane sulfonate, N-tetradecyl-N,N-dimethyl-3-ammonio-1- propane sulfonate and the like.

In the liposome B, these lipids and surfactants are used alone or in combination, and preferably they are used in combination. As the combination in the case where they are used in combination, for example, a combination of two or more components selected from a hydrogenated soybean phosphatidylcholine, a polyethylene glycolated phospholipid and cholesterol, a combination of two or more components selected from distearoyl phosphatidylcholine, a polyethylene glycolated phospholipid and cholesterol, a combination of EPC and DOTAP, a combination of EPC, DOTAP and a polyethylene glycolated phospholipid, a combination of EPC, DOTAP, cholesterol and a polyethylene glycolated phospholipid, and the like can be exemplified.

Further, the liposome B may contain a membrane stabilizer such as a sterol including cholesterol, an antioxidant such as tocopherol or the like as needed.

Examples of the lipid assembly include a spherical micelle, a spherical reversed micelle, a sausage-shaped micelle, a sausage-shaped reversed micelle, a plate-shaped micelle, a plate-shaped reversed micelle, hexagonal I, hexagonal II and an associated product comprising two or more lipid molecules.

Examples of the emulsion particles include oil-in-water (o/w) emulsion particles such as a fat emulsion, an emulsion which comprises a nonionic surfactant and soybean oil, lipid emulsion and lipid nanosphere, water-in-oil-in-water (w/o/w) emulsion particles and the like.
Examples of the polymer include natural polymers such as albumin, dextran, chitosan, dextran sulfate and DNA, synthetic polymers such as poly-L-lysine, polyethyleneimine, polyaspartic acid, a copolymer of styrene with maleic acid, a copolymer of isopropylacrylamide with acrylpyrrolidone, PEG-modified dendrimer, polylactic acid, polylactic acid polyglycolic acid and polyethylene glycolated polylactic acid, a salt thereof and the like.

Here, the salt of the polymer includes, for example, a metal salt, an ammonium salt, an acid addition salt, an organic amine addition salt, an amino acid addition salt and the like. Examples of the metal salt include alkali metal salts such as a lithium salt, a sodium salt and a potassium salt, alkaline earth metal salts such as a magnesium salt and a calcium salt, an aluminum salt, a zinc salt and the like. Examples of the ammonium salt include salts of ammonium, tetramethylammonium and the like. Examples of the acid addition salt include inorganates such as a hydrochlorate, a sulfate, a nitrate and a phosphate, and organates such as an acetate, a maleate, a fumarate and a citrate. Examples of the organic amine addition salt include addition salts of morpholine, piperidine and the like, and examples of the amino acid addition salt include addition salts of glycine, phenylalanine, aspartic acid, glutamic acid, lysine and the like.

Examples of the metal colloid include metal colloids including gold, silver, platinum, copper, rhodium, silica, calcium, aluminum, iron, indium, cadmium, barium, lead and the like.

Examples of the fine particles preparation include a microsphere, a microcapsule, a nanocrystal, lipid nanoparticles, a polymeric micelle and the like.

The lead particle in the present disclosure preferably contains a lipid derivative or a fatty acid derivative of one or more substance(s) selected from, for example, sugars and water-soluble polymers or a surfactant or the like. The lipid derivative or the fatty acid derivative of one or more substance(s) selected from sugars and water-soluble polymers or the surfactant may be used as a constituent component of the lead particle or may be used by adding it to the constituent components of the lead particle.

In the present disclosure preferred examples of the lipid derivative or the fatty acid derivative of one or more substance(s) selected from sugars and water-soluble polymers or the surfactant include a glycolipid or a lipid derivative or a fatty acid derivative of a water-soluble polymer, and more preferred examples thereof include a lipid derivative or a fatty acid derivative of a water-soluble polymer. The lipid derivative or the fatty acid derivative of one or more substance(s) selected from sugars and water-soluble polymers or the surfactant is preferably a substance having a dual character that a part of the molecule has a property of binding to another constituent component(s) of the lead particle due to, for example, hydrophobic affinity, electrostatic interaction or the like, and other part has a property of binding to a solvent used in the production of the lead particle due to, for example, hydrophilic affinity, electrostatic interaction or the like.

Examples of the lipid derivative or the fatty acid derivative of a sugar include those comprising a sugar such as sucrose, sorbitol or lactose, and any of the lipid illustrated in the above-mentioned definition of the lead particle or a fatty acid such as stearic acid, palmitic acid, myristic acid or lauric acid bonded to each other and the like.

Examples of the lipid derivative or the fatty acid derivative of a sugar include the glyceroglycolipids and the sphingoglycolipids illustrated in the above-mentioned definition of the lead particle and the like.
Examples of the lipid derivative or the fatty acid derivative of a water-soluble polymer include those comprising polyethylene glycol, polyglycerol, or a derivative thereof and any of the lipid illustrated in the above-mentioned definition of the lead particle or a fatty acid such as stearic acid, palmitic acid, myristic acid or lauric acid bonded to each other and the like. More preferably, a lipid derivative or a fatty acid derivative of a polyethylene glycol derivative or a polyglycerol derivative can be exemplified, and further more preferably, a lipid derivative or a fatty acid derivative of a polyethylene glycol derivative can be exemplified.

Examples of the lipid derivative or the fatty acid derivative of a polyethylene glycol derivative include a polyethylene glycolated lipid [specifically, polyethylene glycol phosphatidyl ethanolamine (more specifically, 1,2-distearoyl-sn-glycero-3-phosphoethanolamine-N-[methoxy-(polyethylene glycol)-2000] (PEG-DSPE) and the like), polyoxyethylene hydrogenated castor oil 60, Cremophor EL and the like], a polyethylene glycol sorbitan fatty acid ester (specifically, polyoxyethylene sorbitan monooleate and the like), a polyethylene glycol fatty acid ester and the like, and more preferred examples include a polyethylene glycolated lipid.

Examples of the lipid derivative or the fatty acid derivative of a polyglycerol derivative include a polyglycerolated lipid (specifically, polyglycerol phosphatidyl ethanolamine and the like), a polyglycerol fatty acid ester and the like, and more preferred examples include a polyglycerolated lipid.

Examples of the surfactant include the surfactants illustrated in the above-mentioned definition of the lead particle, a polyethylene glycol alkyl ether and the like, and preferred examples thereof include
polyoxyethylene polyoxypropylene glycol, a glycerol fatty acid ester, a polyethylene glycol alkyl ether and the like.

Further, the lead particle preferably has a positive electric charge. The "positive electric charge" as used herein includes an electric charge, surface polarization and the like which generate electrostatic attraction to an electric charge in the above-mentioned RNA, intramolecular polarization and the like. In order for the lead particle to have a positive electric charge, the lead particle preferably contains a cationic substance, more preferably contains a cationic lipid.

The cationic substance to be contained in the lead particle is a substance exhibiting a cationic nature, however, even if it is an amphoteric substance having both cationic group and anionic group, the relative electronegativity varies depending on the pH, bonding to another substance or the like, therefore, the amphoteric substance can be classified into a cationic substance as the case may be. These cationic substances may be used as a constituent component of the lead particle or may be used by adding it to the constituent components of the lead particle.

Examples of the cationic substance include the cationic substances among those illustrated in the above-mentioned definition of the lead particles (specifically, a cationic lipid, a cationic surfactants (the same definition as above), a cationic polymer and the like), a protein or a peptide with which a complex can be formed at a pH equal to or less than an isoelectric point, and the like.

Examples of the cationic lipid include DOTAP, DOTMA, DOSPA, DMRIE, DORIE, DC-Chol and the like.

Examples of the cationic polymer include poly-L-lysine, polyethyleneimine, polyfect, chitosan and the like.

The protein or the peptide with which a complex can be formed at a pH equal to or less than an isoelectric point is not particularly limited as long as it is a protein or a peptide with which a complex can be formed at a pH equal to or less than the isoelectric point of the substance. Examples thereof include albumin, orosomucoid, globulin, fibrinogen, pepsin, ribonuclease T1 and the like.

The lead particle in the present invention can be produced by or in accordance with a known production method, and a lead particle produced by any production method can be used. For example, in the production of lead particle comprising, as a constituent component, liposome B, which is one type of the lead particle, a known liposome preparation method can be applied. As the known liposome preparation method, for example, liposome preparation method by Bangham, et al. [see "Journal of Molecular Biology" (J. Mol. Biol.), Vol. 13, pp. 238-252 (1965)], an ethanol injection method [see "Journal of Cell Biology" (J. Cell Biol.), Vol. 66, pp. 621-634 (1975)], a French press method [see "FEBS Letters" (FEBS Lett.), Vol. 99, pp. 210-214. (1979)], a freeze-thaw method [see "Archives of Biochemistry and Biophysics" (Arch. Biochem. Biophys.), Vol. 212, pp. 186-194 (1981)], a reverse phase evaporation method [see "Proceedings of the National Academy of Science United States of America" (Proc. Natl. Acad. Sci. USA), Vol. 75, pp. 4194-4198 (1978)], a pH gradient method (see, for example, Japanese Patent No. 2,572,554, Japanese Patent No. 2,659,136, etc.) and the like. As a solution for dispersing liposome B in the production of the liposome B, for example, water, an acid, an alkali, any of various buffers, a physiological saline solution, an amino acid infusion or the like can be used. Further, in the production of the liposome B, it is also possible to add an antioxidant such as citric acid, ascorbic acid, cysteine or ethylenediamine tetraacetic acid (EDTA), an isotonic agent such as glycerol, glucose, sodium chloride or the like. Further, the liposome can also be produced by dissolving a lipid or the like in, for example, an organic solvent such as ethanol, distilling off the solvent, adding a physiological saline solution or the like and stirring the mixture by shaking, thereby forming the liposome B.

Further, surface improvement of the liposome can be optionally carried out using, for example, a nonionic surfactants (the same definition as above), a cationic surfactants (the same definition as above), an anionic surfactants (the same definition as above), a polymer, a polyoxyethylene derivative or the like, and such a surface-improving liposome is also used as a constituent component of the lead particles in the present invention [see "Stealth Liposome", edited by D. D. Lasic and F. Martin, CRC Press Inc., USA, pp. 93-102 (1995)]. Examples of the polymer include dextran, pullulan, mannan, amylopectin, hydroxyethylstarch and the like. Examples of the polyoxyethylene derivative include Polysorbate 80, Pluronic F68, polyoxyethylene hydrogenated castor oil 60, polyoxyethylene lauryl alcohol, PEG-DSPE and the like. The surface improvement of the liposome can be employed as one of the methods of incorporating lipid derivative or a fatty acid derivative of one or more substance(s) selected from sugars, peptides, nucleic acids and water-soluble polymers or a surfactant in the lead particles.

An average particle diameter of the liposome B can be freely selected upon demand. Examples of a method of adjusting the average particle diameter include an extrusion method and a method in which a large multilamellar liposome vesicle (MLV) is mechanically pulverized (specifically using Manton-gaulin, a microfluidizer or the like) (see "Emulsion and Nanosuspensions for the Formulation of Poorly Soluble Drugs", edited by R. H. Muller, S. Benita and B. Bohm, Scientific Publishers, Stuttgart, Germany, pp. 267-294, 1998) and the like.

In addition, the method of producing a complex obtained by combining two or more substances selected from, for example, a lipid assembly, liposome B, an emulsion particle, a polymer, a metal colloid, a fine particle preparation and the like, which constitute the lead particle, may be, for example, a production method in which, for example, a lipid, a polymer or the like are only mixed in water. At this time, a granulation step, a sterilization step or the like can be further added as needed. Further, it is also possible to perform the formation of the complex in any of various solvents such as acetone and ether.

As for the size of the lead particle in the present invention, an average particle diameter is preferably several nanometers to several tens micrometers, more preferably 10 nm to 1000 nm, further more preferably 50 nm to 300 nm.
Examples of the constituent component of the lipid membrane in the present invention include the lipids and the surfactants illustrated in the above-mentioned definition of the lead particle and the like, and preferred examples thereof include a neutral lipid among the lipids and the surfactants, more preferred examples thereof include a phospholipid, and further more preferred examples thereof include EPC. Further, the constituent component of the lipid membrane is preferably soluble in a polar organic solvent, and it is preferred that the polar organic solvent can be contained in a liquid at such a concentration that the constituent components of the lipid membrane are dispersible and the complex particles are dispersible. The neutral lipid as used herein means a lipid excluding the cationic lipid and cationic surfactant illustrated in the cationic substance in the case where the lead particle has a positive electric charge described above and the anionic lipid and the anionic surfactant illustrated in the adhesion-competitive agent described below among the lipids and surfactants, and preferred examples of the neutral lipid include a phospholipid, a glyceroglycolipid, a sphingoglycolipid and the like.

Examples of the polar organic solvent in the present invention include alcohols such as methanol, ethanol, n-propanol, 2-propanol, n-butanol, 2-butanol, and tert-butanol, glycols such as glycerol, ethylene glycol and propylene glycol, polyalkylene glycols such as polyethylene glycol and the like, and preferred examples thereof include ethanol.

Further, examples of the lipid to be used in the lipid membrane include a synthetic lipid and the like. Examples of the synthetic lipid include fluorinated phosphatidylcholine, fluorinated surfactants, dialkylammonium bromide and the like. These may be used alone or in combination with another lipid or the like. Further, the lipid membrane contains a lipid derivative, a fatty acid derivative or an aliphatic hydrocarbon derivative, of a water-soluble substance, preferably contains the above-mentioned lipid derivative or the fatty acid derivative of a water-soluble polymer, more preferably contains the above-mentioned polyethylene glycolated phospholipid, and most preferably contains polyethylene glycol phosphatidyl ethanolamine.

Examples of the lipid derivative, the fatty acid derivative or the aliphatic hydrocarbon derivative of a water-soluble substance in the present invention include the above-mentioned lipid derivative or fatty acid derivative of one or more substance(s) selected from sugarsand water-soluble polymers or the aliphatic hydrocarbon derivative of one or more substance(s) selected from sugars and water-soluble polymers, preferred examples thereof include a glycolipid or, a lipid derivative or a fatty acid derivative of a water-soluble polymer, and more preferred examples thereof include a lipid derivative or a fatty acid derivative of a water-soluble polymer.

Examples of the aliphatic hydrocarbon derivative of a water-soluble substance include those comprising a water-soluble substance and, for example, an alcoholic residue of a long-chain aliphatic alcohol, polyoxy polypropylene alkyl, an glycerin fatty acid ester or the like bonded to each other.

Examples of the aliphatic hydrocarbon derivative of a sugar include an aliphatic hydrocarbon derivative of a sugar such as sucrose, sorbitol or lactose.

Examples of the aliphatic hydrocarbon derivative of a water-soluble polymer include an aliphatic hydrocarbon derivative of a polyethylene glycol derivative or a polyglycerol derivative, and more preferred examples thereof include an aliphatic hydrocarbon derivative of a polyethylene glycol derivative.

In the case where the lead particle is a fine particle comprising liposome B as a constituent component, a substance which comprises complex particles comprising the liposome B and the above-mentioned RNA as constituent components and a lipid membrane for coating the complex particles becomes liposome A, which is classified into liposome in a narrow sense based on its structure. Even if the lead particle is different from a fine particle comprising the liposome B as a constituent component, the lead particle is coated with a lipid membrane, therefore, the resulting substance is classified into liposome in a wide sense. In the present invention, it is more preferred that the constituent component of the lead particle is also liposome.

The complex particles comprising the lead particle and the RNA as constituent components in the present invention can be produced by adhering or encapsulating the RNA to or into the lead particle after or concurrently with the production of the lead particle. Further, liposome A can be produced by coating the complex particles with the lipid membrane after or concurrently with the production of the complex particles. The liposome A can be produced by or in accordance with a known production method described in, for example, Published Japanese translation of a PCT international application No. 2002-508765, Published Japanese translation of a PCT international application No. 2002-501511, "Biochimica et Biophysica Acta", Vol. 1510, pp. 152-166 (2001), and International Publication No. WO 02/28367, or can be produced by a production method including a step of dispersing the complex particles and coating layer components in a liquid which contains a polar organic solvent in which the coating layer components are soluble at a concentration at which the complex particles are not dissolved and the coating layer components are present in a dispersed state after the complex particles are produced by adhering or encapsulating the RNA to or into the lead particle, and a step of coating the complex particles with the coating layer components.

As a preferred production method of the liposome in the composition of the present invention, the following production method including a step of producing complex particles comprising as constituent components a lead particle and the RNA (step 1) and a step of coating the complex particles with a lipid membrane (step 2 or step 3) can be exemplified.

Step 1) Step of producing complex particles comprising as constituent components a lead particle and the RNA
Lead particles are dispersed in a solvent such as water, the RNA is dispersed or dissolved by mixing so as to be contained in the liquid in which the lead particles are dispersed, and the RNA is adhered to the lead particles. In the step 1, in order to suppress the aggregation of the lead particles, the lead particles are preferably lead particles comprising an aggregation-suppressing substance, and more preferably the lead particles contain as the aggregation-suppressing substance, the above-mentioned lipid derivative or fatty acid derivative of one or more substance(s) selected from sugars, peptides, nucleic acids and water-soluble polymers or the surfactant. Further, in the case where the lead particles have a positive electric charge, the RNA and an adhesion-competitive agent are allowed to coexist in the liquid in which the lead particles are dispersed, and the adhesion-competitive agent may be adhere to the lead particles as well as the RNA. Further, also in the case where the lead particles are lead particles comprising the aggregation-suppressing substance, in order to further suppress the aggregation of the lead particles, the adhesion-competitive agent may be used. In the combination of the lead particles and the RNA, it is preferred that a combination in which the complex particles are dispersible in the liquid containing a polar organic solvent is selected, and it is more preferred that the solubility of the complex particles in the polar organic solvent is lower than that of the constituent components of the lipid membrane to be used in the step 2 or 3. It is further more preferred that a combination in which the polar organic solvent can be contained in a liquid at such a concentration that the constituent components of the lipid membrane are dispersible and the complex particles are dispersible is selected.

Examples of the adhesion-competitive agent include an anionic substance and the like, and the anionic substance includes a substance electrostatically adhered to the constituent components of the lead particles due to the electrostatic attraction by an electric charge, intramolecular polarization or the like in the molecule. The anionic substance as the adhesion-competitive agent is a substance exhibiting an anionic nature, however, even if it is an amphoteric substance having both cationic group and anionic group, the relative electronegativity varies depending on the pH, binding to another substance or the like, therefore, the amphoteric substance can be classified into an anionic substance as the case may be.

Examples of the anionic substance include the anionic substances among those illustrated in the above-mentioned definition of the lead particles (specifically, an anionic lipid, an anionic surfactant (the same definition as above), an anionic polymer and the like), a protein or a peptide, with which a complex can be formed at a pH equal to or greater than an isoelectric point, a nucleic acid and the like. Preferred examples thereof include one or more substance(s) selected from dextran sulfate, sodium dextran sulfate, chondroitin sulfate, sodium chondroitin sulfate, hyaluronic acid, chondroitin, dermatan sulfate, heparan sulfate, heparin, keratan sulfate, dextran fluorescein anionic, and the like.

Examples of the anionic lipid include phosphatidylserine, phosphatidylglycerol, phosphatidylinositol, phosphatidic acid and the like.
Examples of the anionic polymer include polyaspartic acid, a copolymer of styrene with maleic acid, a copolymer of isopropylacrylamide with acrylpyrrolidone, PEG-modified dendrimer, polylactic acid, polylactic acid polyglycolic acid, polyethylene glycolated polylactic acid, dextran sulfate, sodium dextran sulfate, chondroitin sulfate, sodium chondroitin sulfate, hyaluronic acid, chondroitin, dermatan sulfate, heparan sulfate, heparin, keratan sulfate, dextran fluorescein anionic and the like.

The protein or the peptide with which a complex can be formed at a pH equal to or greater than an isoelectric point is not particularly limited as long as it is a protein or a peptide with which a complex can be formed at a pH equal to or greater than the isoelectric point of the substance. Examples thereof include albumin, orosomucoid, globulin, fibrinogen, histone, protamine, ribonuclease, lysozyme and the like.

Examples of the nucleic acid as an anionic substance include DNA, RNA, plasmid, siRNA, ODN and the like. It may have any length and any sequence as long as it does not exhibit a physiological activity.

The adhesion-competitive agent is preferably electrostatically adhered to the constituent components of the lead particles, and is preferably a substance with a size which does not allow the crosslinking formation to aggregate the constituent components of the lead particles even if the substance is adhered to the constituent components of the lead particles, or a substance having in its molecule, a moiety which is adhered to the constituent components and a moiety which repels the adhesion and suppresses the aggregation of the lead particles.
More specifically, the step 1 can be carried out, for example, in a production method including a step of producing a liquid in which lead particles comprising an aggregation- suppressing substance are dispersed, and a step of dispersing or dissolving the RNA so as to be contained in the liquid in which the lead particles are dispersed (for example, a step of adding the RNA to the liquid in which the lead particles are dispersed and dispersing or dissolving the RNA therein, a step of adding a liquid in which the RNA is dispersed or dissolved to the liquid in which the lead particles are dispersed or the like). Here, specific examples of the complex particles obtained by the step of dispersing or dissolving the RNA so as to be contained in the liquid in which the lead particles are dispersed, include complex particles formed by adhering the RNA to fine particles comprising as a constituent component, liposome B comprising the cationic lipid, complex particles formed by adhering the RNA to fine particles comprising as a constituent component, a lipid assembly comprising the cationic lipid, and complex particles formed by adhering the RNA to fine particles comprising as a constituent component, a polymer comprising a cationic polymer such as poly-L-lysine. Further, the step of dispersing or dissolving the RNA so as to be contained in the liquid in which the lead particles are dispersed is preferably a step of further incorporating the adhesion-competitive agent in the liquid in which the RNA is dispersed or dissolved and adding the resulting liquid to the liquid in which the lead particles are dispersed. In this case, the complex particles are produced by adhering both of the RNA and the adhesion-competitive agent to the lead particles, and the production can be carried out by further suppressing aggregation of the lead particles during the production of the complex particles and aggregation of the complex particles after the production.

The ratio of the lead particles to the liquid in which the lead particles are dispersed is not particularly limited as long as the RNA can be adhered to the lead particles, however, it is preferably 1 µg/mL to 1 g/mL, more preferably 0.1 to 500 mg/mL.

### Step 2) Step of coating complex particles with lipid membrane (1)

Liposome A can be produced by, for example, a production method including a step of preparing a liquid (liquid A) containing a polar organic solvent in which the complex particles obtained in the step 1 are dispersed and the constituent components of the lipid membrane are dissolved, and a step of coating the complex particles with the lipid membrane by reducing the ratio of the polar organic solvent in the liquid A. In this case, the liposome A is obtained in the form of a dispersion (liquid B). The solvent in the liquid A is a solvent which contains a polar organic solvent at such a concentration that the constituent components of the lipid membrane are soluble and the complex particles are dispersible. In the liquid B in which the ratio of the polar organic solvent to the liquid A is reduced, the constituent components of the lipid membrane are dispersible and the complex particles are also dispersible. In the case where the solvent in the liquid A is a liquid mixture of a polar organic solvent and a solvent different from a polar organic solvent, for example, by adding a solvent comprising a solvent different from a polar organic solvent mixable with the polar organic solvent (liquid C), and/or selectively removing the polar organic solvent by distillation by evaporation, semipermeable membrane separation, fractional distillation or the like, the ratio of the polar organic solvent can be reduced. Here, the liquid C is a solvent comprising a solvent different from a polar organic solvent, and may also contain a polar organic solvent as long as the ratio of the polar organic solvent is lower than that of the polar organic solvent contained in the liquid A.

Examples of the solvent different from a polar organic solvent in the step 2 include water, liquid carbon dioxide, a liquid hydrocarbon, a halogenated carbon, a halogenated hydrocarbon and the like, and preferred examples thereof include water. Further, the liquid A and the liquid C may contain an ion, a buffer component or the like.

The combination of a polar organic solvent with a solvent different from a polar organic solvent is preferably a combination of solvents that are mixable with each other and can be selected by considering the solubility of the complex particles and the constituent components of the lipid membrane in the solvents in the liquid A and the liquid B, and the liquid C. On the other hand, the complex particles preferably have a low solubility in any of the solvents in the liquid A and the liquid B, and the liquid C, and also preferably have a low solubility in any of a polar organic solvent and a solvent different from a polar organic solvent. The constituent components of the lipid membrane preferably have a low solubility in the solvent in the liquid B and the liquid C, and preferably have a high solubility in the solvent in the liquid A, and preferably have a high solubility in a polar organic solvent and preferably have a low solubility in a solvent different from a polar organic solvent. Here, the complex particles having a low solubility means that the elution of each component contained in the complex particles such as the lead particles, RNA and adhesion-competitive agent in the solvent is low, and even if the respective solubilities of the components are high, it is sufficient that the elution of each component becomes low due to the binding or the like between the respective components. For example, even in the case where the solubility of any of the components contained in the lead particles in the solvent in the liquid A is high, if the lead particles have a positive electric charge, and an electrostatic bond is formed due to an electric charge, intramolecular polarization or the like in the RNA, and the solubility of the component(s) in the solvent in the liquid A becomes low, the elution of the components in the complex particles is suppressed, whereby the solubility of the complex particles in the solvent in the liquid A can be lowered That is, if the lead particles have a positive electric charge, the elution of the components of the complex particles is suppressed in the production of the liposome A, and an effect of improving the productivity and yield is imparted.

The ratio of the polar organic solvent in the liquid A is not particularly limited as long as it is a ratio at which the constituent components of the lipid membrane are soluble and the complex particles are dispersible, and varies depending on the solvent or the complex particles to be used, the type of constituent components of the lipid membrane or the like. However, it is preferably 30 v/v% or more, more preferably 60 to 90 v/v%. Further, the ratio of the polar organic solvent in the liquid B is not particularly limited as long as the liquid B contains the polar organic solvent at a concentration lower than the liquid A and it is a ratio at which the constituent components of the lipid membrane are dispersible and the complex particles are also dispersible, however, it is preferably 50 v/v% or less.

The step of preparing the liquid A may be a step of preparing the liquid A by adding the polar organic solvent, the complex particles and the constituent components of the lipid membrane, or the polar organic solvent, the complex particles, the constituent components of the lipid membrane and the solvent different from the polar organic solvent in any order as long as the complex particles are not dissolved. Preferably, a step of preparing the liquid A by preparing a liquid (liquid D) containing a polar organic solvent in which the complex particles are dispersed, preparing a liquid (liquid E) in which the constituent components of the lipid membrane are dissolved in a solvent containing a polar organic solvent that is the same as or different from the polar organic solvent in the liquid D1 and mixing the liquid D and the liquid E can be exemplified. When the liquid A is prepared by mixing the liquid D and the liquid E, it is preferred to mix them gradually.

### Step 3) Step of coating complex particles with lipid membrane (2)

Liposome A can be produced by a production method including a step of dispersing the complex particles obtained in the step 1 and the constituent components of the lipid membrane in a liquid (liquid F) which contains a polar organic solvent in which the constituent components of the lipid membrane are soluble at a concentration at which the complex particle are not dissolved and the constituent components of the lipid membrane are present in a dispersed state. In this case, the liposome A can be obtained in a state of a dispersion. The solvent in the liquid F is a solvent which contains a polar organic solvent in which the constituent components of the lipid membrane are soluble, and in which in a solvent comprising the polar organic solvent, wherein the polar organic solvent can be contained in a liquid at such a concentration that the constituent components of the lipid membrane are dispersible and the complex particles are dispersible.

As a method of preparing the liquid F, any embodiment can be employed. For example, the liquid F may be prepared by preparing a dispersion of complex particles and a solution or a dispersion of the constituent components of the lipid membrane and mixing both liquids, or the liquid F may be prepared by preparing either one of the dispersions of the complex particles and the constituent components of the lipid membrane, and adding and dispersing the other remaining complex particles or constituent components of the lipid membrane in the form of a solid in the resulting dispersion. In the case where a dispersion of the complex particles and a solution or a dispersion of the constituent components of the lipid membrane are mixed, a dispersion medium of the complex particles may contain a polar organic solvent in advance, further, the constituent components of the coating lipid membrane may be dissolved or dispersed therein, and a solvent or a dispersion medium of the constituent components of the lipid membrane may be a liquid containing a polar organic solvent or a liquid composed only of a polar organic solvent. On the other hand, in the case where the dispersions of either one of the complex particles and the constituent components of the lipid membrane is prepared, and the other remaining complex particles or constituent components of the lipid membrane in the form of a solid are added to the resulting dispersion, the resulting dispersion is a liquid containing a polar organic solvent. Incidentally, in the case where the complex particles are not dissolved and the constituent components of the lipid membrane are dispersed after the liquid F is prepared, a polar organic solvent may be added in a concentration range of the polar organic solvent in which the complex particles are not dissolved and the constituent components of the lipid membrane are dispersed, or the organic solvent may be removed or the concentration thereof may be reduced. On the other hand, in the case where the complex particles are not dissolved and the constituent components of the lipid membrane are dissolved after the liquid F is prepared, the polar organic solvent may be removed or the concentration thereof may be reduced in a concentration range of the polar organic solvent in which the complex particles are not dissolved and the constituent components of the lipid membrane are dispersed. Alternatively, the complex particles and the constituent components of the lipid membrane are mixed in a solvent different from a polar organic solvent in advance, and a polar organic solvent may be added thereto in a concentration range of the polar organic solvent in which the complex particles are not dissolved and the constituent components of the lipid membrane are dispersed. In this case, the complex particles and the constituent components of the lipid membrane are separately dispersed in solvents different from a polar organic solvent, and both dispersions are mixed, and then, a polar organic solvent may be added thereto, or either one of the complex particles and the constituent components of the lipid membrane are dispersed in a solvent different from a polar organic solvent, and the other remaining complex particles or constituent components of the lipid membrane in the form of a solid are added to the resulting dispersion, and then a polar organic solvent may be added thereto. Further, it is preferred to include a step of letting a liquid, in which the complex particles and the constituent components of the lipid membrane are dispersed and a polar organic solvent is contained, stand or mixing the liquid for a time sufficient to coat the complex particles with the lipid membrane. The time for letting the liquid stand or mixing the liquid after the complex particles and the constituent components of the lipid membrane are dispersed in the liquid comprising the polar organic solvent is not limited as long as it is not completed immediately after the complex particles and the constituent components of the lipid membrane are dispersed in the liquid comprising the polar organic solvent, however, it can arbitrarily set depending on the constituent components of the lipid membrane or the type of the liquid comprising the polar organic solvent, and it is preferably set to a time which keeps the yield of the obtained liposome A constant, for example, 3 seconds to 30 minutes.

Examples of the solvent different from a polar organic solvent in the liquid F include those illustrated in the solvent different from a polar organic solvent in the step 2, and preferred examples thereof include water.

The ratio of the polar organic solvent in the liquid F is not particularly limited as long as only the requirement that both of the complex particles and the constituent components of the lipid membrane are dispersed is met, and varies depending on the solvent or the complex particles to be used, the type of the constituent components of the lipid membrane or the like. However, it is preferably 1 to 80 vol%, more preferably 10 to 60 vol%, more preferably 20 to 50 vol%, and the most preferably 30 to 40 vol%.

In the present invention, the description of the constituent components of the lipid membrane being soluble in a polar organic solvent include a case in which the constituent components of the lipid membrane have a property of being dissolved in a polar organic solvent, a case in which the constituent components of the lipid membrane have a property of being dissolved in a polar organic solvent with the help of a solubilizer or the like, a case in which the constituent components of the lipid membrane have a property capable of being emulsified or formed into an emulsion by forming aggregates, micelles or the like in a polar organic solvent and the like. Further, the description of the constituent components of the lipid membrane being dispersible includes a state in which the whole of the constituent components of the lipid membrane form aggregates, micelles or the like and are emulsified or formed into an emulsion, a state in which a part of the constituent components of the lipid membrane form aggregates, micelles or the like and are emulsified or formed into an emulsion, and the rest of the components are dissolved, a state in which a part of the constituent components of the lipid membrane form aggregates, micelles or the like and are emulsified or formed into an emulsion, and the rest of the components are precipitated and the like. Incidentally, the description of the constituent components of the lipid membrane being dissolved does not include a state in which the whole of the constituent components of the lipid membrane form aggregates, micelles or the like and are emulsified or formed into an emulsion.

In the present invention, the description of the complex particles being dispersed means a state in which the complex particles are suspended or emulsified or formed into an emulsion, and includes a state in which a part of the complex particles are suspended or emulsified or formed into an emulsion, and the rest of the particles are dissolved, a state in which a part of the complex particles are emulsified or formed into an emulsion, and the rest of the particles are precipitated and the like. The description of the complex particles being not dissolved is the same as the above-mentioned definition of the complex particles being dispersed.

The concentration of the complex particles in the liquid containing a polar organic solvent to be used in the method of producing liposome A according to the present disclosure is not particularly limited as long as it allows the complex particles to be coated with the lipid membrane, however, it is preferably 1 µg/mL to 1 g/mL, more preferably 0.1 to 500 mg/mL. Further, the concentration of the components of the lipid membrane to be used is not particularly limited as long as it allows the complex particles to be coated, however, it is preferably 1 µg/mL to 1 g/mL, more preferably 0.1 to 400 mg/mL.
The ratio of the lipid membrane to the liposome A of the present disclosure is preferably 1:0.1 to 1:1000, more preferably 1:1 to 1:10 in ratio by weight.

Further, as for the size of the liposome A of the present disclosure, an average particles diameter is preferably 300 nm or less, more preferably 200 nm or less. Specifically, for example, an injectable size is preferred.

Further, the liposome A obtained above can be modified with a substance such as a protein including an antibody and the like, a saccharide, a glycolipid, an amino acid, a nucleic acid or any of various low-molecular compounds and polymers, and such coated complex particles obtained by modification is included in the liposome A. For example, in order to apply to targeting, it is possible that the liposome A obtained above is further subjected to a surface modification of the lipid membrane using a protein such as an antibody, a peptide, a fatty acid or the like [see "Stealth Liposome", edited by D. D. Lasic and F. Martin, CRC Press Inc., USA, pp. 93-102, (1995)]. Further, surface improvement can also be optionally carried out to the liposome A using, for example, a lipid derivative, a fatty acid derivative or an aliphatic hydrocarbon derivative, of a water-soluble substance. The lipid derivative, the fatty acid derivative and the aliphatic hydrocarbon derivative of a water-soluble substance to be used in the surface modification are the same definition as the lipid derivative, the fatty acid derivative and the aliphatic hydrocarbon derivative of a water-soluble substance as the constituting components of the lipid membrane.

By administering the composition of the present invention to a mammal, the RNA can be delivered to a tissue or an organ including an expression site of a target gene, and for example, an RNA capable of suppressing the expression of KLF5 gene and a gene whose transcription is activated by KLF5 can be introduced into a mammalian cell in vivo, and the expression of KLF5 gene, a gene whose transcription is activated by KLF5 and the like can be suppressed. By the composition of the present invention, for example, the expression of KLF5 gene and a gene whose transcription is activated by KLF5 is suppressed, and the proliferation of smooth muscle and angiogenesis can be suppressed, therefore, the composition of the present invention can be used as an active ingredient of a therapeutic agent or a preventive agent for a cardiovascular disease such as arteriosclerosis, restenosis occurring after coronary intervention or cardiac hypertrophy, cancer or the like. Further, in the case where the target gene of the RNA is a gene associated with a tumor or an inflammation or a gene associated with angiogenesis, for example, it is possible to treat a tumor by administering the composition as a therapeutic agent for the tumor, or it is possible to treat an inflammation by administering the composition as a therapeutic agent for the inflammation. Further, the composition of the present invention can also be used as a tool for acquiring POC (proof of concept) in an in vivo screening system.

The composition of the present invention can be used as a preparation intended for stabilization of the RNA in a living body component such as a blood component (for example, blood, gastrointestinal tract or the like), reduction of side effects, increase in the accumulation property of a drug in a target organ such as a tumor, improvement in absorption of a drug orally or via mucous membrane or the like.
In the case where the composition of the present invention is used as a drug preparation, it is preferred that an administration route that is most effective for treatment is used. Examples of the administration route include parenteral administration routes such as intraoral administration, tracheobronchial administration, intrarectal administration, subcutaneous administration, intramuscular administration, and intravenous administration and oral administration routes. Preferred examples thereof include intravenous administration and intramuscular administration, and more preferred examples thereof include intravenous administration.

As a preparation suitable for intravenous administration or intramuscular administration, for example, an injection can be exemplified, and it is also possible to use the dispersion of the liposome A prepared by the above-mentioned method as it is in the form of, for example, an injection or the like. However, it can also be used after removing the solvent from the dispersion by, for example, filtration, centrifugation or the like, or after lyophilizing the dispersion or the dispersion supplemented with an excipient such as mannitol, lactose, trehalose, maltose or glycine.

In the case of an injection, it is preferred that an injection is prepared by mixing, for example, water, an acid, an alkali, any of various buffers, a physiological saline solution, an amino acid infusion or the like with the dispersion of the liposome A or the liposome A obtained by removing the solvent or lyophilization. Further, it is possible to prepare an injection by adding an antioxidant such as citric acid, ascorbic acid, cysteine or EDTA, an isotonic agent such as glycerol, glucose or sodium chloride or the like. Further, it can also be cryopreserved by adding a cryopreservation agent such as glycerol.

Further, the liposome A may be formulated into an oral preparation such as a capsule, a tablet or a granule by granulating along with an appropriate excipient or the like, drying or the like.

Examples of the liposome of the present disclosure include liposome which comprises complex particles comprising as constituent components a lead particle and the RNA, and a lipid membrane for coating the complex particles, constituent components of the lipid membrane can be solved in a polar organic solvent, the polar organic solvent can be contained in a liquid at such a concentration that the constituent components of the lipid membrane are dispersible and the complex particles are dispersible; and liposome which comprises complex particles comprising as constituent components a lead particle comprising a cationic lipid and the above-mentioned RNA, and a lipid membrane for coating the complex particles, wherein the lipid membrane contains, as constituent components, a neutral lipid and a lipid derivative, a fatty acid derivatives or an aliphatic hydrocarbon derivative, of a water-soluble substance.

Hereinafter, the present invention will be specifically described with reference to Examples and Reference examples. However, the present invention is not limited to these Examples and Reference examples.

### Reference example 1: Preparation of siRNA 1

As the sequence of siRNA which is capable of suppressing the expression of KLF5 gene, from the sequence of mouse KLF5 cDNA (GenBank Accession No. NM_009769; SEQ ID NO: 41), 11 partial sequences that satisfy the following 2 requirements were selected: (a) a sequence consisting of 21 nucleotides that begins with AA; and (b) a GC content between 20% and 80%. Such sequences were preferably selected from sequences, which are located in a coding region (the sequence at nucleotides 167-1507 of SEQ ID NO: 41) and further located 75 nucleotides or more downstream of the start codon (the sequence at nucleotides 167-169 of SEQ ID NO: 41), and which have a GC content between 40% and 60%. The positions of the selected sequences in SEQ ID NO: 41 and GC contents thereof were shown in Table 1. Sequences obtained by substituting T with U in 19 nucleotides excluding AA at the 5'-terminus thereof in the selected sequences were shown in SEQ ID numbers: 1 to 11.

[Table 1]

**Table 1**

| Sequence selected | Sequence position | GC content | Produced RNA sequence | SEQ ID NO | siRNA No. |
|---|---|---|---|---|---|
| AACATGAACGTCTTCCTCCCT | 537-556 | 48% (10/21) | CAUGAACGUCUUCCUCCCUTT | 17 | No. 1 |
| | | | AGGGAGGAAGACGUUCAUGTT | 18 | |
| AAATTCTGCCACTCTGCC | 1156-1176 | 48% (10/21) | AUUUACCUGCCACUCUGCCUU | 19 | No. 2 |
| | | | GGCAGAGUGGCAGGUAAAUUU | 20 | |
| AAGGAGTAACCCGGATCTGGA | 1216-1236 | 52% (11/21) | GGAGUAACCCGGAUCUGGAUU | 21 | No. 3 |
| | | | UCCAGAUCCGGGUUACUCCUU | 22 | |
| AAAAGCTCACCTGAGGACTCA | 1303-1323 | 48% (10/21) | AAGCUCACCUGAGGACUCAUU | 23 | No. 4 |
| | | | UGAGUCCUCAGGUGAGCUUUU | 24 | |
| AATCCCCAGACCGTCCATGCC | 151-171 | 62% (13/21) | UCCCCAGACCGUCCAUGCCUU | 25 | No. 5 |
| | | | GGCAUGGACGGUCUGGGGGUU | 26 | |
| AACGCTGCGCCCACCCGCCTG | 1515-1535 | 76% (16/21) | CGCUGCGCCCACCCGCCUGUU | 27 | No. 6 |
| | | | CAGGCGGGUGGGCGCAGCGUU | 28 | |
| AAATGGAGAAGTATCTGACCC | 405-425 | 43% (9/21) | AUGGAGAAGUAUCUGACCCUU | 29 | No. 7 |
| | | | GGGUCAGAUACUUCUCCAUUU | 30 | |
| AAAGTATAGACGAGACAGTGC | 463-483 | 43% (9/21) | AGUAUAGACGAGACAGUGCUU | 31 | No. 8 |
| | | | GCACUGUCUCGUCUAUACUUU | 32 | |
| AAACCAGACGGCAGTAATGGA | 874-894 | 48% (10/21) | ACCAGACGGCAGUAAUGGAUU | 33 | No. 9 |
| | | | UCCAUUACUGCCGUCUGGCUU | 34 | |
| AAGCTCAGAGCCTGGAAGTCC | 2048-2068 | 57% (12/21) | GCUCAGAGCCUGGAAGUCCUU | 35 | No. 10 |
| | | | GGACUUCCAGGCUCUGAGCUU | 36 | |
| AAGCCGTTCCAGTGCATGGTG | 1424-1444 | 57% (12/21) | GCCGUUCCAGUGCAUGGUGUU | 37 | No. 11 |
| | | | CACCAUGCACUGGAACGGCUU | 38 | |

11 types of double-stranded RNAs (hereinafter referred to as siRNA Nos. 1 to 11, respectively), which have a sequence shown in any one of SEQ ID numbers: 1 to 11 and a sequence complementary thereto, wherein UU or dTdT have been added to the 3'-terminus of each of the sequences, were prepared as follows. The sequences of the sense strand and antisense strand of each of siRNA Nos. 1 to 11 are shown in Table 1 (SEQ ID numbers: 17 to 38). siRNA No. 1 was prepared as follows. Namely, chemical synthesis of two RNAs shown in SEQ ID numbers: 17 and 18 was carried out by Japan Bio Services, Co., Ltd., and the thus obtained RNAs were annealed with each other. SiRNA Nos. 2 to 11 were prepared by in vitro transcription using Silencer™ siRNA Construction Kit (manufactured by Ambion). DNA to be used for the production of a template for the in vitro transcription was prepared by Hokkaido System Science Co., Ltd.

### Reference example 2: Preparation of siRNA 2

With regard to the siRNA Nos. 2 to 4 and 7 to 11 obtained in Reference example 1, mouse KLF5 cDNA and human KLF5 cDNA were aligned based on the sequence homology, whereby a human sequence that corresponds to a sequence selected in mouse. In Table 2, sequences consisting of 21 nucleotides on mouse KLF5 cDNA used as bases for the design, and the positions thereof on SEQ ID NO: 41, sequences consisting of 21 nucleotides on human cDNA corresponding to the above mouse sequences, and the positions thereof on SEQ ID NO: 42, and SEQ ID numbers indicating RNA sequences obtained by excluding AA at the 5'-terminus from the above human sequences were shown. Since siRNA No. 5 and No. 6 were based on the sequence of a non-coding region, human sequences corresponding thereto were not indicated.

[Table 2]

**Table 2**

| siRNA No. | Mouse KLFS cDNA | | Human KLF5 cDNA | | |
|---|---|---|---|---|---|
| | Sequence | Position | Corresponding sequence | Position | SEQ ID NO |
| No. 2 | AAATTTACCTGCCACTCTGCC | 1156-1176 | AAATTTACCCACCACCCTGCC | 1334-1354 | 12 |
| No. 3 | AAGGAGTAACCCGGATCTGGA | 1216-1236 | AAGGAGTAACCCCGATTTGGA | 1394-1414 | 13 |
| No. 4 | AAAAGCTCACCTGAGGACTCA | 1303-1323 | AAAAGCTCACCTGAGGACTCA | 1481-1501 | 4 |
| No. 7 | AAATGGAGAAGTATCTGACCC | 405-425 | AAATGGAGAAGTATCTGACAC | 583-603 | 14 |
| No. 8 | AAAGTATAGACGAGACAGTGC | 463-483 | AAAGTATAGACGAGACAGTGC | 641-661 | 8 |
| No. 9 | AAACCAGACGGCAGTAATGGA | 874-894 | AAATCAGACAGCAGCAATGGA | 1040-1060 | 15 |
| No. 10 | AAGCTCAGAGCCTGGAAGTCC | 2048-2068 | AAGCTCAGAGCCTGGAAGTCC | 1226-1246 | 10 |
| No. 11 | AAGCCGTTCCAGTGCATGGTG | 1424-1444 | AAGCCCTTCCAGTGCGGGGTG | 1602-1622 | 16 |

### Example 1

DOTAP (manufactured by Avanti Polar Lipids Inc.), PEG-DSPE (manufactured by NOF Corporation, hereinafter the same shall apply) and distilled water were mixed such that the ratio of DOTAP/PEG-DSPE/distilled water was 30 mg/12 mg/mL, and the mixture was stirred by shaking with a vortex mixer. The obtained dispersion was passed, at room temperature, through a polycarbonate membrane filter of 0.4 µm pore size (manufactured by Whatman) for 4 times and through a polycarbonate membrane filter of 0.1 µm pore size (manufactured by Whatman) for 10 times and then through a polycarbonate membrane filter of 0.05 µm pore size (manufactured by Whatman) for 24 times, whereby lead particles were prepared. To 0.5 mL of the obtained dispersion of lead particles, 0.25 mL of an 8 mg/mL aqueous solution of siRNA No. 4 of Reference example 1 or 2 was added, and 1 mL of ethanol was further added thereto, whereby complex particles were prepared. To the obtained dispersion of complex particles, 0.25 mL of a solution in which EPC (manufactured by NOF Corporation) and PEG-DSPE, both of which were the constituent components of the lipid membrane, were dissolved in ethanol such that the ratio of EPC/PEG-DSPE/ethanol was 120 mg/25 mg/mL was added, and then, 23 mL of distilled water was gradually added thereto to adjust the concentration of ethanol to be 5 vol% or less, whereby liposome was prepared. The obtained dispersion of liposome was subjected to ultracentrifugation (110, 000 × g at 25°C for 1 hour) and the supernatant was removed. A physiological saline solution was added thereto to disperse the residue again, whereby liposome dispersion was obtained (a PEG-DSPE ethanol solution). 50 parts by weight of PEG-DSPE based on 120 parts by weight of EPC was dissolved in a small amount (about 1/25 volume of the liposome dispersion) of ethanol. The liposome dispersion and the PEG-DSPE ethanol solution were separately heated at 70°C for 2 minutes. Then, the liposome dispersion was added to the PEG-DSPE ethanol solution and mixed. Then, the resulting mixture was heated at 70°C for 2 minutes and cooled with water, whereby a preparation was obtained.
The preparation was prepared in 3 lots.

When the average particle diameters of liposome were measured by a dynamic light scattering method (A model ELS-800, Otsuka Electronics Co., Ltd.), they were 102, 103 and 95 nm, respectively.

### Example 2

To 0.5 mL of a dispersion of lead particles obtained in the same manner as in Example 1, 0.25 mL of an 8 mg/mL aqueous solution of each of siRNA No. 1 to 3 and 5 to 16 of Reference example 1 or 2 was added, and 1 mL of ethanol was further added thereto, whereby complex particles of each of the siRNA No. 1 to 3 and 5 to 16 were obtained. The obtained dispersion of the complex particles was subjected to the same procedure as in Example 1, whereby liposome encapsulating each of the siRNA No. 1 to 3 and 5 to 16 was obtained. The obtained liposome was subjected to the same procedure as in Example 1, whereby a preparation of each of the siRNA No. 1 to 3 and 5 to 16 was obtained.

### Comparative example 1

The siRNA in Example 1 was altered into scrambled KLF5-siRNA (sense strand: 5'-GGU ACA CAU GUG CAC ACA C - dTdT-3'; antisense strand: 5'-GUG UGU GCA CAU GUG UAC C-dTdT-3', Hokkaido System Science Co., Ltd.), and a preparation was obtained in the same manner. The preparation was prepared in 3 or more lots. Incidentally, scrambled KLF5-siRNA is an RNA which does not contain a sequence of KLF5-mRNA and a sequence complementary to the sequence.
The average particle diameters of liposome were measured by a dynamic light scattering method (Zetasizer NanoZS, manufactured by Malvern Instruments). In one example, it was 88 nm.

### Comparative example 2

By dissolving siRNA No. 4 (KLF5 siRNA) of Reference example 1 or 2 in a physiological saline solution, a 0.5 mg/mL aqueous solution was prepared.

### Comparative example 3

By dissolving the same scrambled KLF5 siRNA as in Comparative example 1 in a physiological saline solution, a 0.5 mg/mL aqueous solution was prepared.

### Test example 1

1 × 10⁶ murine LL/2 lewis lung carcinoma cells ("The Journal of Biological Chemistry", Vol. 278, pp. 34598-34604, 2003) were subcutaneously administered to a mouse (BL6J mouse, male, 5 weeks of age). LL/2 is significantly affected by angiogenesis in terms of its growth, therefore, it is used in screening for an angiogenesis inhibitor. From the time when the cells were immobilized (1 to 2 days after the subcutaneous administration), the preparation obtained in Example 1 was administered through the tail vein on consecutive days. The administered preparation was diluted in advance with a phosphate-buffered saline solution to give a final concentration of the total lipid of 5 mg/mL, and the dose to the mouse was set to 150 µg/shot/mouse/day in terms of siRNA.

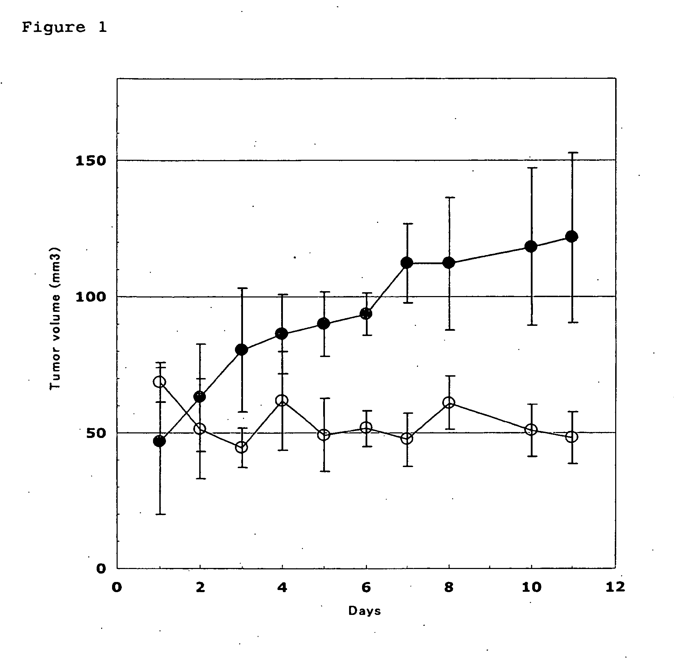

The volume of the tumor transplanted into the mouse was measured over time and the growth of the tumor was measured. The results are shown in Fig. 1.

Further, the tumor was excised from the mouse 10 days after the administration, and angiogenesis around the tumor cells was observed, and also CD31 immunostaining and hematoxylin-eosin staining were carried out by the following method using a section of tissue around the tumor, and observation was carried out with a microscope.

### CD31 immunostaining

After the tumor fixed in methanol was embedded in paraffin, a section with a thickness of 5 µm was prepared. After the section was deparaffinized, blocking was carried out at room temperature for 10 minutes by adding 0.5% goat serum. After the blocking solution was sucked, an antimouse CD31 antibody (100-fold dilution) (manufactured by Pharmingen Co.) was added thereto and the section was let stand at room temperature for 2 hours. After the section was washed with PBS, a biotin-conjugated anti-rat Ig antibody (200-fold dilution) (manufactured by DAKO Inc.) was added thereto and the section was allowed to stand at room temperature for 1 hour. After the section was washed with PBS, ABC-AP Kit AK-5000 (manufactured by Vector Laboratories Inc.) was added thereto and the section was allowed to stand at room temperature for 30 minutes. After the section was washed with PBS, Vector Red SK-5100 (manufactured by Vector Laboratories Inc.) was added thereto and the section was allowed to stand at room temperature for 30 minutes thereby to allow a color to develop. After the section was washed with water, it was subjected to post-staining with a hematoxin staining solution, followed by dehydration, and then, observation was carried out with a microscope.

### Hematoxylin-eosin staining

After the tumor fixed in methanol was embedded in paraffin, a section with a thickness of 5 µm was prepared. After the section was deparaffinized, it was washed with flowing water, and then, passed through distilled water. A hematoxylin staining solution was added thereto and the section was allowed to stand for 5 minutes. Then, the section was washed with water, and separation was carried out by adding 0.5% hydrochloric acid solution. The section was washed with flowing water for 5 minutes to allow a color to develop, and passed through distilled water. An eosin staining solution was added thereto and the section was allowed to stand for 3 minutes. Separation was carried out by adding 95% ethanol, followed by dehydration, and then, observation was carried out with a microscope.

According to Fig. 1, in the group administered with the preparation obtained in Example 1, the growth of cancer cells were suppressed compared with the non-treatment group. Further, in the observation of the periphery of tumor cells 10 days after the administration, in the group administered with the preparation obtained in Example 1, the size of the tumor itself was smaller than that of the non-treatment group, and moreover, extensive necrosis of the central area of the tumor was observed. Further, in the microscopic observation of tumor tissue section subjected to hematoxylin-eosin staining and CD31 immunostaining in the group administered with the preparation obtained in Example 1, a decrease in the vascular endothelial cells observed around and in the tumor was observed, compared with the non-treatment group. It was apparent that angiogenesis was suppressed in the group administered with the preparation obtained in Example 1.

That is, it has become apparent that by suppressing the antiangiogenesis effect and the growth of tumor cells, the composition of the present invention can be efficiently delivered to the vicinity of a tumor, for example, in the case where KLF5-siRNA is intravenously administered to a mammal, and the expression of KLF5 gene can be suppressed.

### Test example 2

1 × 10⁶ murine LL/2 lewis lung carcinoma cells were subcutaneously administered to a mouse (C57BL/6jjcl mouse, male, 6 weeks of age, CLEA Japan, Inc.) in the axillary area. Between 2 and 8 days after the tumor cell injection, a preparation comprising 50 µg of siRNA (Example 1 or Comparative example 1), an siRNA solution comprising 50 µg of siRNA (Comparative example 2 or 3), or a physiological saline solution was administered through the tail vein on consecutive days.

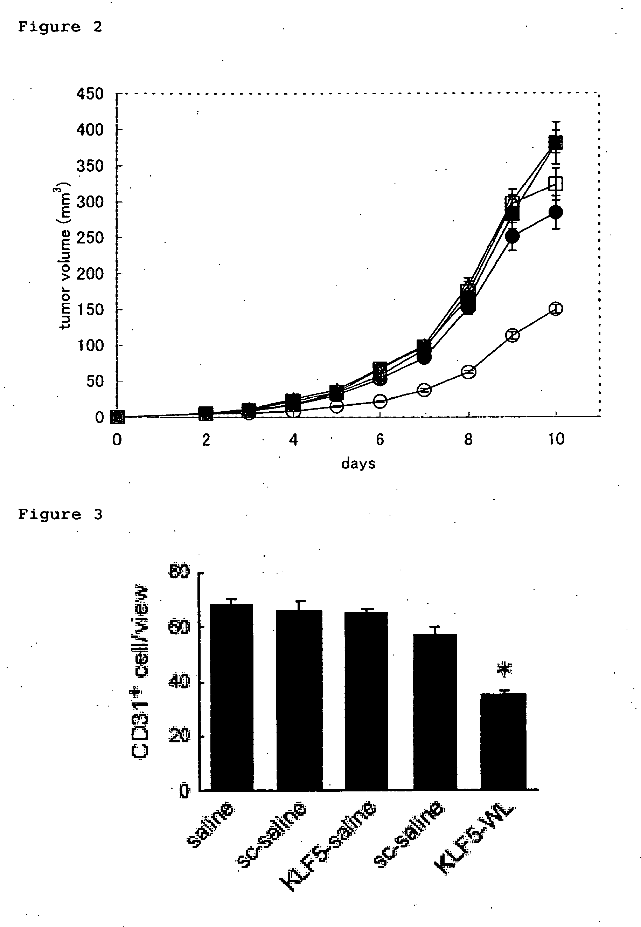

The volume of the tumor transplanted into the mouse was measured over time. The tumor volume was calculated using the formula: (major axis of tumor) × (minor axis)² × 1/2, and the significant difference was assayed by the Tukey-kramer method. The results are shown in Fig. 2.

Further, the tumor was excised from the mouse 10 days after the administration, and angiogenesis around the tumor cells was observed, and also a section of tissue around the tumor was subjected to CD31 immunostaining by the following method, and CD31 structures observed in the tissue section were counted. Two visual fields were studied per one tumor, and the structures were counted for 5 tumors each, and the average thereof was shown. The significant difference was assayed by the Tukey-kramer method. The results are shown in Fig. 3.

### CD31 immunostaining

The tumor was excised and fixed in a 95% methanol solution, and then, embedded in paraffin. After the tumor was sliced to have a thickness of 5 µm, it was deparaffinized, and then, an anti-CD31 polyclonal antibody (manufactured by Pharmingen Co., USA) diluted to 100-fold was allowed to act on at room temperature for 120 minutes. After the CD31 antibody was washed, a biotinylated anti-rat IgG (manufactured by DAKO Inc. USA) diluted to 200-fold was allowed to act on at room temperature for 60 minutes. After washing was carried out, an avidin-HRP conjugate (manufactured by Vector Laboratories Inc., USA) was allowed to act on at room temperature for 30 minutes. After washing was carried out, a color was allowed to develop with diaminobenzine (manufactured by Sigma Co., USA).

According to Fig. 2, in only the group administered with the preparation obtained in Example 1, a significant effect of suppressing tumor growth was observed. Further, according to Fig. 3, in the group administered with the preparation obtained in Example 1, the number of the brown colored CD31 structures showing angiogenesis was small, and angiogenesis in the tumor was inhibited.

### Test example 3

1 × 10⁶ murine LL/2 lewis lung carcinoma cells were subcutaneously administered to a mouse (C57BL/6jjcl mouse, male, 6 weeks of age, CLEA Japan, Inc.) in the axillary area. A preparation comprising 50 µg of siRNA (Example 1 or Comparative example 1), an siRNA solution comprising 50 µg of siRNA (Comparative example 2 or 3), or a physiological saline solution was administered. 36 hours after the administration, the tumor was excised, and the mRNA expression level and the KLF5 level were measured by the following methods.

### Measurement of mRNA expression level

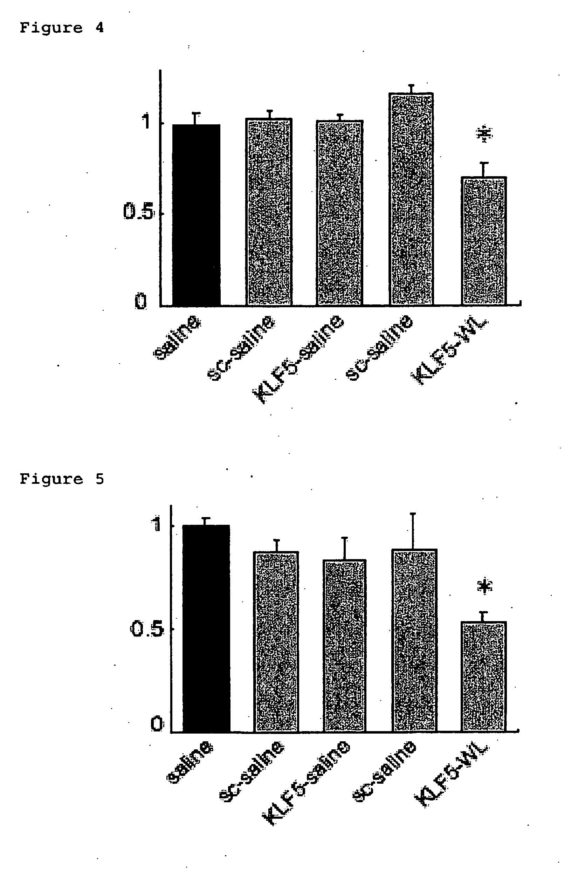

With the use of RNeasy Fibrous tissue kit (manufactured by Qiagen Inc.), the excised tumor cells were dissolved using zirconia balls and a mixer mill (MM300, Qiagen), and purification was carried out, whereby RNA was obtained. With the use of SuperScript First-Strand Synthesis Kit (manufactured by Invitrogen Inc., USA), cDNA was obtained by reverse transcription using a random primer from 1 µg of the resulting RNA. The resulting cDNA was amplified using Hot Star Taq^{™} (manufactured by Qiagen Inc.) and specific primers. At this time, a ribosomal 18S RNA primer was used as an internal standard. The amplification cycles were carried out as follows: 95°C for 15 minutes and 45 cycles of 94°C for 30 seconds, 53°C for 30 seconds, and 72°C for 30 seconds. The primer sequences were as follows: 5' - GGTTGCACAAAAGTTTATAC-3', and 5'-GGCTTGGCGCCTGTGTGCTTCC-3'. The mRNA expression level was determined using ABI PRISM^{™} 7900HT (manufactured by Applied Biosystems Inc., USA) and QuantiTect SYBR Green PCR kit (manufactured by Qiagen Inc.). Standardization was carried out by using QuantumRNA^{™} Classic 18S ribosomal RNA primer (manufactured by Ambion Inc., USA) as an internal standard. The significant difference was assayed by the Tukey-kramer method. The results are shown in Fig. 4.

### Measurement of KLF5 level

The excised tumor cells were soaked in an appropriate amount of a dissolution solution (150 mmol/L NaCl, 20 mmol/L Tris, pH 7.5, 0.1% Nonidet P-40, 0.1% sodium laulyl sulfate, 0.5% sodium deoxycholate, 0.1 mmol/L DTT, a protease inhibitor), and dissolved using zirconia balls and a mixer mill (MM300, Qiagen), and a centrifuged supernatant was obtained. The protein level was measured using Dc protein assay reagent (Bio-Rad, USA). 10 µg of protein was subjected to a heat treatment at 95°C for 15 minutes and then 10% polyacrylamide gel electrophoresis. The electrophoresed protein was transferred to a nitrocellulose membrane (manufactured by Amersham Inc. USA), and a blocking treatment (in 5% skimmed milk while shaking) was carried out. Then, the membrane was treated in a 1000-fold dilution of an anti-KLF5 monoclonal antibody (manufactured by Kyowa Hakko Kogyo K.K.) for 1 hour while shaking. After the membrane was washed, it was treated with a 2000-fold dilution of HRP-labeled anti-rat IgG (manufactured by Amersham Inc.). After washing was carried out, a color was allowed to develop by using a fluorescence coloring agent (ECLplus, manufactured by Amersham Inc.), and the Western blotting band was determined using an image analysis software (Image Quant, manufactured by Amersham Inc.). One group consisted of 3 mice, and only the group administered with KLF5 siRNA consisted of 5 mice and determination was carried out. The significant difference was assayed by the Tukey-kramer method. The results are shown in Fig. 5.

According to Fig. 4, only in the group administered with the preparation obtained in Example 1, suppression of the production of KLF5 mRNA was observed. According to Fig. 5, only in the group administered with the preparation obtained in Example 1, suppression of the production of KLF5 was observed.

### Reference test example 1

1 × 10⁶ murine LL/2 lewis lung carcinoma cells were subcutaneously administered to a mouse (C57BL/6jjcl mouse, male, 5 to 6 weeks of age) in the axillary area. Between 2 and 8 days after the tumor cell injection, an siRNA solution comprising 1 µg of siRNA (Comparative example 2 or 3), or a physiological saline solution was directly administered to the tumor on consecutive days.

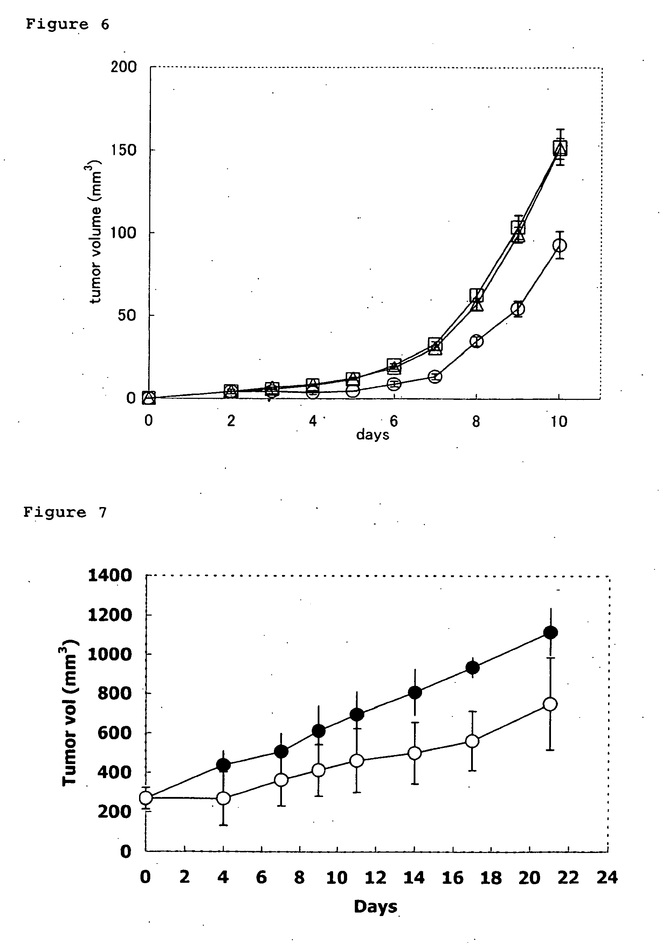

The volume of the tumor transplanted into the mouse was measured over time. The tumor volume was calculated using the formula: (major axis of tumor) × (minor axis)² × 1/2, and the significant difference was assayed by the Tukey-kramer method. The results are shown in Fig. 6.
According to Fig. 6, a tendency of suppressing the growth of tumor was observed in the group administered with the solution of KLF5 siRNA obtained in Comparative example 1, however, a significant difference was not observed.

Further, the tumor was excised from the mouse 10 days after the administration, and a section of tissue around the tumor was subjected to CD31 immunostaining in the same manner as in Test example 2, and observation was carried out. An apparent difference in CD31 showing angiogenesis was not observed in both groups.

### Example 3

The siRNA in Example 1 was altered into bcl2-siRNA (sense strand: 5'-GUGAAGUCAACAUGCCUGC-dTdT-3': antisense strands: 5'-GCAGGCAUGUUGACUUCAC-dTdT-3', Dharmacon Inc. or Hokkaido System Science Co., Ltd.), and a preparation was obtained in the same manner. The preparation was prepared in 3 or more lots.
The average particle diameters of liposome were measured by a dynamic light scattering method (Zetasizer NanoZS, manufactured by Malvern Instruments). In one example, it was 110 nm.

### Comparative example 4

The siRNA in Example 1 was altered into GL3-siRNA (sense strand: 5'-CUUACGCUGAGUACUUCGA-dTdT-3'; antisense strand: 5'-UCGAAGUACUCAGCGUAAG-dTdT-3', Dharmacon Inc. or Hokkaido System Science Co., Ltd.), and a preparation was obtained in the same manner. The preparation was prepared in 3 or more lots. Incidentally, GL3-siRNA is an RNA which does not contain a sequence of bcl2-mRNA and a sequence complementary to the sequence.
The average particle diameters of liposome were measured by a dynamic light scattering method (Zetasizer NanoZS, manufactured by Malvern Instruments). In one example, it was 99 nm.

### Comparative example 5

By dissolving the same bcl2-siRNA as in Example 3 in a physiological saline solution, a 0.5 mg/mL aqueous solution was prepared.

### Test example 4

1 × 10⁷ human prostate carcinoma cells DU145 (ATCC No. HTB-81) were subcutaneously administered to a nude mouse (BALB/cAJcl-nu, male, 5 weeks of age, CLEA Japan, Inc.). The tumor volume was measured 17 days after the subcutaneous administration, and a nude mouse in which the tumor volume reached 150 to 350 mm³ was selected. Then, the preparation obtained in Example 3 was administered to the selected mouse through the tail vein. The administration was carried out for 5 consecutive days, followed by a recovery period of 2 days, and then, administration was further carried out for 5 consecutive days.
The administered preparation was diluted in advance with a physiological saline solution to give a final concentration of the siRNA of 0.75 mg/mL, and the dose to the mouse was set to 150 µg/shot/mouse/day in terms of siRNA.
The volume of the tumor transplanted into the mouse was measured over time and the growth of the tumor was measured. The results are shown in Fig. 7.
According to Fig. 7, in the group administered with the preparation obtained in Example 3, the growth of tumor cells was suppressed compared with the non-treatment group.

### Test-example 5

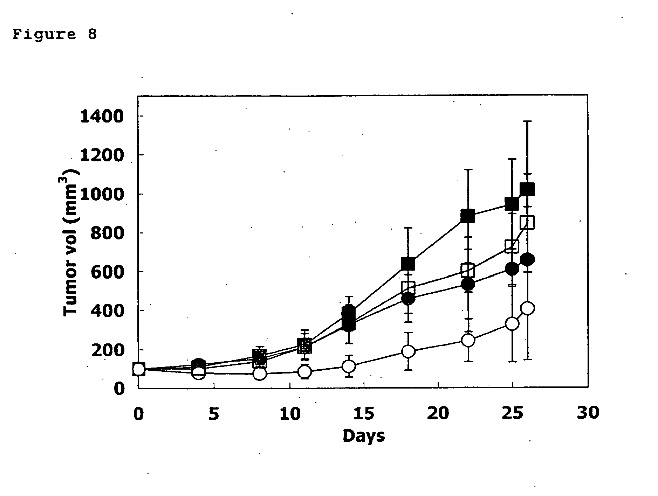

1 × 10⁶ human prostate carcinoma cells PC-3 (ATCC No. CRL-1435) were subcutaneously administered to a nude mouse (BALB/cAJcl-nu, male, 6 weeks of age, CLEA Japan, Inc.). The tumor volume was measured 6 days after the subcutaneous administration, and a nude mouse in which the tumor volume reached 80 to 120 mm³ was selected. Then, the preparation obtained in Example 3, Comparative example 4 or 5 was administered to the selected mouse through the tail vein. The administration was carried out for 5 consecutive days, followed by a recovery period of 2 days, and then, administration was further carried out for 5 consecutive days.
The administered preparation was diluted in advance with a physiological saline solution to give a final concentration of the siRNA of 0.75 mg/mL, and the dose to the mouse was set to 150 µg/shot/mouse/day in terms of siRNA.
The volume of the tumor transplanted into the mouse was measured over time and the growth of the tumor was measured. The results are shown in Fig. 8.
According to Fig. 8, in the group administered with the preparation obtained in Example 3, the growth of tumor cells was suppressed compared with the non-treatment group, the groups administered with the preparation obtained in Comparative example 4, and the solution obtained in Comparative example 5.

### Industrial Applicability

By administering the composition of the present invention comprising liposome encapsulating an RNA comprising a sequence consisting of 15 to 30 contiguous nucleotides of a target gene mRNA and a sequence complementary to the sequence to a mammal or the like, the expression of the target gene can be suppressed.

### [SEQUENCE LISTING FREE TEXT]

SEQ ID NO: 1, Inventors: YAMAUCHI MASAHIRO; TOTTORI TSUNEAKI; ISHIHARA ATSUSHI; YAGI NOBUHIRO; KATO YASUKI
SEQ ID NO: 17 siRNA No. 1 sense strand
SEQ ID NO: 18 siRNA No. 1 antisense strand
SEQ ID NO: 19 siRNA No. 2 sense strand
SEQ ID NO: 20 siRNA No. 2 antisense strand
SEQ ID NO: 21 siRNA No. 3 sense strand
SEQ ID NO: 22 siRNA No. 3 antisense strand
SEQ ID NO: 23 siRNA No. 4 sense strand
SEQ ID NO: 24 siRNA No. 4 antisense strand
SEQ ID NO: 25 siRNA No. 5 sense strand
SEQ ID NO: 26 siRNA No. 5 antisense strand
SEQ ID NO: 27 siRNA No. 6 sense strand
SEQ ID NO: 28 siRNA No. 6 antisense strand
SEQ ID NO: 29 siRNA No. 7 sense strand
SEQ ID NO: 30 siRNA No. 7 antisense strand
SEQ ID NO: 31 siRNA No. 8 sense strand
SEQ ID NO: 32 siRNA No. 8 antisense strand
SEQ ID NO: 33 siRNA No. 9 sense strand
SEQ ID NO: 34 siRNA No. 9 antisense strand
SEQ ID NO: 35 siRNA No. 10 sense strand
SEQ ID NO: 36 siRNA No. 10 antisense strand
SEQ ID NO: 37 siRNA No. 11 sense strand
SEQ ID NO: 38 siRNA No. 11 antisense strand
SEQ ID NO: 39 SEAP siRNA sense strand
SEQ ID NO: 40 SEAP siRNA antisense strand

## Claims

1. A composition comprising an RNA-encapsulating liposome, wherein the RNA comprises a sequence consisting of 15 to 30 contiguous nucleotides of a target gene mRNA and a sequence complementary to the sequence, and the RNA has an action of suppressing the expression of the target gene utilizing RNA interference (RNAi), and the liposome comprises:
complex particles comprising as constituent components:
(i) a lead particle comprising a cationic lipid and
(ii) the RNA, and
a lipid membrane for coating the complex particles, and the lipid membrane contains, as constituent components, a neutral lipid and a lipid derivative, a fatty acid derivative or an aliphatic hydrocarbon derivative, of a water-soluble substance,
wherein the lipid derivative, fatty acid derivative or aliphatic hydrocarbon derivative of a water-soluble substance is selected from the group consisting of a polyethylene glycolated lipid, a polyethylene glycol sorbitan fatty acid ester, a polyethylene glycol fatty acid ester, a polyglycerolated lipid and a polyglycerol fatty acid ester,
wherein the liposome is capable of reaching a tissue or an organ including an expression site of the target gene and has a size that allows intravenous administration.

2. The composition according to claim 1, wherein the target gene is a gene associated with a tumor or an inflammation.

3. The composition according to any one of claims 1 or 2, wherein the target gene is a gene associated with angiogenesis.

4. The composition according to claim 1, wherein the mRNA is KLF5 mRNA.

5. The composition according to claim 1, wherein the mRNA is either human or mouse KLF5 mRNA.

6. The composition according to any one of claims 4 or 5, wherein the RNA is a double-stranded RNA, which has a strand of a sequence consisting of 15 to 30 contiguous nucleotides of KLF5 mRNA and a strand of a sequence complementary to the sequence, except that 1 to 6 nucleotides, which are the same or different, are added to the 3'-terminus of each of the strands in the same or a different manner.

7. The composition according to any one of claims 4 or 5, wherein the RNA is an RNA, which has a hairpin structure, and in which an RNA having a sequence consisting of 15 to 30 contiguous nucleotides of KLF5 mRNA is ligated to an RNA having a sequence complementary to the sequence via a spacer oligonucleotide, and 1 to 6 nucleotides, which are the same or different, are added to the 3'-terminus thereof.

8. The composition according to any one of claims 4 or 5, wherein the RNA is an RNA selected from the group consisting of the following (a) to (c):
(a) a double-stranded RNA, which has a strand of a sequence shown in any one of SEQ ID numbers: 2 to 16 and a strand of a sequence complementary to the sequence, except that 2 to 4 members, which are the same or different, selected from uridylic acids and deoxythymidylic acids are added to the 3'-terminus of each of the strands in the same or a different manner;
(b) an RNA, which has a hairpin structure, and in which an RNA having a sequence shown in any one of SEQ ID numbers: 2 to 16 is ligated to an RNA having a sequence complementary to the sequence via a spacer oligonucleotide having 2 uridylic acids or deoxythymidylic acids at the 5'-terminus thereof, and 2 to 4 members, which are the same or different, selected from uridylic acids and deoxythymidylic acids are added to the 3'-terminus thereof; and
(c) a double-stranded RNA, which has a strand of a sequence shown in any one of SEQ ID numbers: 2 to 11 and a strand of a sequence complementary to the sequence, except that 2 uridylic acids are added to the 3'-terminus of each of the strands.

9. The composition according to claim 1, wherein the mRNA is bcl2 mRNA.

10. The composition according to any one of claims 1 to 9, wherein constituent components of the lipid membrane are dissoluble in a polar organic solvent, and wherein the constituent components of the lipid membrane and the complex particles are dispersible in the polar organic solvent.

11. The composition according to any one of claims 1 to 9, wherein the RNA-encapsulating liposome is obtainable by a production method including the steps of:
preparing a liquid A containing a polar organic solvent and water, in which the constituent components of the lipid membrane are dissolved and the complex particles are dispersed, and
adding water to the liquid A or selectively removing the polar organic solvent by distillation, evaporation, semipermeable membrane separation or fractional distillation from the liquid A, whereby the complex particles are coated with the lipid membrane.

12. The composition according to any one of claims 1 to 9, wherein the RNA-encapsulating liposome is obtainable by a production method including the steps of:
preparing a dispersion of complex particles and a solution or a dispersion of the constituent components of the lipid membrane, and
mixing both liquids to prepare liquid F, wherein the liquid F contains a polar organic solvent, and wherein
the constituent components of the lipid membrane and the complex particles are dispersed, whereby the complex particles are coated with the lipid membrane.

13. The composition according to any one of claims 10 to 12, wherein the polar organic solvent is an alcohol.

14. The composition according to any one of claims 10 to 12, wherein the polar organic solvent is ethanol.

15. The composition according to any one of claims 1 to 14, wherein the cationic lipid is one or more compounds selected from N-[1-(2,3-dioleoylpropyl)]-N,N,N-trimethylammonium chloride, N-[1-(2,3-dioleoylpropyl)]-N,N-dimethylamine, N-[1-(2,3-dioleyloxypropyl)-N,N,N-trimethylammonium chloride, N-[1-(2,3-ditetradecyloxypropyl)]-N,N-dimethyl-N-hydroxyethylammonium bromide and 3β-[N-(N'N'-dimethylaminoethyl)carbamoyl cholesterol.

16. The composition according to any one of claims 1 to 15, wherein the lipid derivative, the fatty acid derivative or the aliphatic hydrocarbon derivative, of a water-soluble substance is polyethylene glycol phosphatidyl ethanolamine.

17. The composition according to any one of claims 1 to 16, wherein the neutral lipid is egg yolk phosphatidylcholine.

## Patentansprüche

1. Zusammensetzung, umfassend ein RNA einkapselndes Liposom, wobei die RNA eine Sequenz, bestehend aus 15 bis 30 benachbarten Nucleotiden einer Targetgen-mRNA, und eine Sequenz, die zu der Sequenz komplementär ist, umfasst und die RNA eine suppressive Wirkung auf die Expression des Targetgens unter Verwendung von RNA-Interferenz (RNAi) hat und das Liposom:
Komplexteilchen, die als Bestandteilkomponenten:
(i) ein Leitpartikel, umfassend ein kationisches Lipid, und
(ii) die RNA umfassen, und
eine Lipidmembran zum Beschichten der Komplexteilchen umfasst und die Lipidmembran als Bestandteilkomponenten ein neutrales Lipid und ein Lipidderivat, ein Fettsäurederivat oder ein aliphatisches Kohlenwasserstoffderivat einer wasserlöslichen Substanz enthält,
wobei das Lipidderivat, Fettsäurederivat oder aliphatische Kohlenwasserstoffderivat einer wasserlöslichen Substanz aus der Gruppe ausgewählt ist, bestehend aus einem polyethylenglycolierten Lipid, einem Polyethylenglycolsorbitanfettsäureester, einem Polyethylenglycolfettsäureester, einem polyglycerolierten Lipid und einem Polyglycerolfettsäureester,
wobei das Liposom ein Gewebe oder ein Organ, das einen Expressionsort des Targetgens enthält, erreichen kann und eine Größe hat, die intravenöse Verabreichung gestattet.

2. Zusammensetzung nach Anspruch 1, wobei das Targetgen ein Gen ist, das mit einem Tumor oder einer Entzündung in Verbindung steht.

3. Zusammensetzung nach einem der Ansprüche 1 oder 2, wobei das Targetgen ein Gen ist, das mit Angiogenese in Verbindung steht.

4. Zusammensetzung nach Anspruch 1, wobei die mRNA KLF5 mRNA ist.

5. Zusammensetzung nach Anspruch 1, wobei die mRNA entweder menschliche oder Maus-KLF5 mRNA ist.

6. Zusammensetzung nach einem der Ansprüche 4 oder 5, wobei die RNA eine doppelsträngige RNA ist, die einen Strang einer Sequenz, bestehend aus 15 bis 30 benachbarten Nucleotiden von KLF5 mRNA, und einen Strang einer Sequenz aufweist, die zu der Sequenz komplementär ist, außer dass 1 bis 6 Nucleotide, die dieselben oder verschiedene sind, an den 3'-Terminus eines jeden der Stränge auf dieselbe oder eine andere Weise addiert sind.

7. Zusammensetzung nach einem der Ansprüche 4 oder 5, wobei die RNA eine RNA ist, die eine Haarnadelstruktur aufweist, und wobei eine RNA mit einer Sequenz, bestehend aus 15 bis 30 benachbarten Nucleotiden von KLF5 mRNA, an eine RNA mit einer Sequenz, die zu der Sequenz komplementär ist, über ein Spaceroligonucleotid ligiert ist und 1 bis 6 Nucleotide, die dieselben oder verschiedene sind, an den 3'-Terminus davon addiert sind.

8. Zusammensetzung nach einem der Ansprüche 4 oder 5, wobei die RNA eine RNA ist, die aus der Gruppe ausgewählt ist, bestehend aus den folgenden (a) bis (c):
(a) einer doppelsträngigen RNA, die einen Strang einer in einer der SEQ ID Nummern: 2 bis 16 gezeigten Sequenz und einen Strang einer Sequenz aufweist, die zu der Sequenz komplementär ist, außer dass 2 bis 4 Glieder, die dieselben oder verschiedene sind, ausgewählt aus Uridylsäuren und Desoxythymidylsäuren, an den 3'-Terminus eines jeden der Stränge auf dieselbe oder eine andere Weise addiert sind;
(b) einer RNA, die eine Haarnadelstruktur aufweiset, und wobei eine RNA mit einer in einer der SEQ ID Nummern: 2 bis 16 gezeigten Sequenz an eine RNA mit einer Sequenz, die zu der Sequenz komplementär ist, über ein Spaceroligonucleotid mit 2 Uridylsäuren oder Desoxythymidylsäuren am 5'-Terminus davon ligiert ist und 2 bis 4 Glieder, die dieselben oder verschiedene sind, ausgewählt aus Uridylsäuren und Desoxythymidylsäuren, an den 3'-Terminus davon addiert sind; und
(c) einer doppelsträngigen RNA, die einen Strang einer in einer der SEQ ID Nummern: 2 bis 11 gezeigten Sequenz und einen Strang einer Sequenz aufweist, die zu der Sequenz komplementär ist, außer dass 2 Uridylsäuren an den 3'-Terminus eines jeden der Stränge addiert sind.

9. Zusammensetzung nach Anspruch 1, wobei die mRNA bcl2 mRNA ist.

10. Zusammensetzung nach einem der Ansprüche 1 bis 9, wobei die Bestandteilkomponenten der Lipidmembran in einem polaren organischen Lösungsmittel löslich sind, und wobei die Bestandteilkomponenten der Lipidmembran und die Komplexteilchen in dem polaren organischen Lösungsmittel dispergierbar sind.

11. Zusammensetzung nach einem der Ansprüche 1 bis 9, wobei das RNA einkapselnde Liposom durch ein Herstellungsverfahren erhältlich ist, das die Schritte:
Herstellen einer Flüssigkeit A, die ein polares organisches Lösungsmittel und Wasser enthält, in der die Bestandteilkomponenten der Lipidmembran gelöst sind und die Komplexteilchen dispergiert sind, und
Zugeben von Wasser zu der Flüssigkeit A oder selektives Entfernen des polaren organischen Lösungsmittels durch Destillation, Eindampfen, Trennung mit einer semipermeablen Membran oder fraktionierte Destillation von der Flüssigkeit A, wobei die Komplexteilchen mit der Lipidmembran beschichtet werden, umfasst.

12. Zusammensetzung nach einem der Ansprüche 1 bis 9, wobei das RNA einkapselnde Liposom durch ein Herstellungsverfahren erhältlich ist, das die Schritte:
Herstellen einer Dispersion von Komplexteilchen und einer Lösung oder einer Dispersion der Bestandteilkomponenten der Lipidmembran und
Mischen der beiden Flüssigkeiten zur Herstellung einer Flüssigkeit F umfasst, wobei die Flüssigkeit F ein polares organisches Lösungsmittel enthält, und wobei die Bestandteilkomponenten der Lipidmembran und die Komplexteilchen dispergiert werden, wodurch die Komplexteilchen mit der Lipidmembran beschichtet werden.

13. Zusammensetzung nach einem der Ansprüche 10 bis 12, wobei das polare organische Lösungsmittel ein Alkohol ist.

14. Zusammensetzung nach einem der Ansprüche 10 bis 12, wobei das polare organische Lösungsmittel Ethanol ist.

15. Zusammensetzung nach einem der Ansprüche 1 bis 14, wobei das kationische Lipid eine oder mehrere Verbindungen, ausgewählt aus N-[1-(2,3-Dioleoylpropyl)]-N,N,N-trimethylammoniumchlorid, N-[1-(2,3-Dioleoylpropyl)]-N,N-dimethylamin, N-[1-(2,3-Dioleoyloxypropyl)-N,N,N-trimethylammoniumchlorid, N-[1-(2,3-Ditetradecyloxypropyl)]-N,N-dimethyl-N-hydroxyethylammoniumbromid und 3β-[N-(N'N'-Dimethylaminoethyl)carbamoylcholesterol ist.

16. Zusammensetzung nach einem der Ansprüche 1 bis 15, wobei das Lipidderivat, das Fettsäurederivat oder das aliphatische Kohlenwasserstoffderivat einer wasserlöslichen Substanz Polyethylenglycolphosphatidylethanolamin ist.

17. Zusammensetzung nach einem der Ansprüche 1 bis 16, wobei das Neutrallipid Eigelbphosphatidylcholin ist.

## Revendications

1. Composition comprenant un liposome d'encapsulation d'ARN, dans laquelle l'ARN comprend une séquence constituée de 15 à 30 nucléotides contigus d'un ARNm de gène cible et une séquence complémentaire à la séquence, et l'ARN a une action de suppression de l'expression du gène cible en utilisant une interférence d'ARN (ARNi), et le liposome comprend :
des particules complexes comprenant en tant que composants constituants :
(i) une particule de tête comprenant un lipide cationique et
(ii) l'ARN, et
une membrane lipidique destinée à revêtir les particules complexes, et la membrane lipidique contient, en tant que composants constituants, un lipide neutre et un dérivé de lipide, un dérivé d'acide gras ou un dérivé d'hydrocarbure aliphatique, d'une substance hydrosoluble, le dérivé de lipide, le dérivé d'acide gras ou le dérivé d'hydrocarbure aliphatique d'une substance hydrosoluble étant choisi dans le groupe constitué d'un lipide au polyéthylène glycol, un ester d'acide gras de polyéthylène glycol sorbitan, un ester d'acide gras de polyéthylène glycol, un lipide polyglycérolé et un ester d'acide gras de polyglycérol,
dans laquelle le liposome est susceptible d'atteindre un tissu ou un organe comprenant un site d'expression du gène cible et a une taille qui permet une administration intraveineuse.

2. Composition selon la revendication 1, dans laquelle le gène cible est un gène associé à une tumeur ou une inflammation.

3. Composition selon l'une quelconque des revendications 1 ou 2, dans laquelle le gène cible est un gène associé à une angiogenèse.

4. Composition selon la revendication 1, dans laquelle l'ARNm est un ARNm KLF5.

5. Composition selon la revendication 1, dans laquelle l'ARNm est un ARNm KLF5 soit humain soit de souris.

6. Composition selon l'une quelconque des revendications 4 ou 5, dans laquelle l'ARN est un ARN à double brin, qui a un brin d'une séquence constituée de 15 à 30 nucléotides contigus d'ARNm KLF5 et un brin d'une séquence complémentaire à la séquence, si ce n'est que 1 à 6 nucléotides, qui sont identiques ou différents, sont ajoutés à la terminaison 3' de chacun des brins de la même manière ou d'une manière différente.

7. Composition selon l'une quelconque des revendications 4 ou 5, dans laquelle l'ARN est un ARN, qui a une structure en épingle à cheveux, et dans laquelle un ARN ayant une séquence constituée de 15 à 30 nucléotides contigus d'ARNm KLF5 est ligaturé à un ARN ayant une séquence complémentaire à la séquence par l'intermédiaire d'un oligonucléotide espaceur, et 1 à 6 nucléotides, qui sont identiques ou différents, sont ajoutés à sa terminaison 3'.

8. Composition selon l'une quelconque des revendications 4 ou 5, dans laquelle l'ARN est un ARN choisi dans le groupe constitué des éléments suivants (a) à (c) :
(a) un ARN à double brin, qui a un brin d'une séquence illustrée dans l'un quelconque des numéros de SEQ ID : 2 à 16 et un brin d'une séquence complémentaire à la séquence, si ce n'est que 2 à 4 membres, qui sont identiques ou différents, choisis parmi les uridyliques et les acides désoxythymidyliques, sont ajoutés à la terminaison 3' de chacun des brins de la même manière ou d'une manière différente ;
(b) un ARN, qui a une structure en épingle à cheveux, et dans lequel un ARN ayant une séquence illustrée dans l'un quelconque des numéros de SEQ ID : 2 à 16 est ligaturé à un ARN ayant une séquence complémentaire à la séquence par l'intermédiaire d'un oligonucléotide espaceur ayant 2 acides uridyliques ou acides désoxythymidyliques à sa terminaison 5', et 2 à 4 membres, qui sont identiques ou différents, choisi parmi les acides uridyliques et acides désoxythymidyliques sont ajoutés à sa terminaison 3' ; et
(a) un ARN à double brin, qui a un brin d'une séquence illustrée dans l'un quelconque des numéros de SEQ ID : 2 à 11 et un brin d'une séquence complémentaire à la séquence, si ce n'est que 2 acides uridyliques sont ajoutés à la terminaison 3' de chacun des brins.

9. Composition selon la revendication 1, dans laquelle l'ARNm est un ARNm bcl2.

10. Composition selon l'une quelconque des revendications 1 à 9, dans laquelle les composants constituants de la membrane lipidique peuvent être dissous dans un solvant organique polaire, et dans laquelle les composants constituants de la membrane lipidique et les particules complexes peuvent être dispersés dans le solvant organique polaire.

11. Composition selon l'une quelconque des revendications 1 à 9, dans laquelle le liposome d'encapsulation d'ARN peut être obtenu par un procédé de production comprenant les étapes consistant à : préparer un liquide A contenant un solvant organique polaire et de l'eau, dans lequel les composants constituants de la membrane lipidique sont dissous et les particules complexes sont dispersées, et
ajouter de l'eau au liquide A ou éliminer de façon sélective le solvant organique polaire par distillation, évaporation, séparation par membrane semi-perméable ou distillation fractionnée du liquide A, moyennant quoi les particules complexes sont revêtues de la membrane lipidique.

12. Composition selon l'une quelconque des revendications 1 à 9, dans laquelle le liposome d'encapsulation d'ARN peut être obtenu par un procédé de production comprenant les étapes consistant à : préparer une dispersion de particules complexes et une solution ou une dispersion des composants constituants de la membrane lipidique, et
mélanger l'un et l'autre liquides pour préparer le liquide F, le liquide F contenant un solvant organique polaire, et dans laquelle
les composants constituants de la membrane lipidique et les particules complexes sont dispersés, moyennant quoi les particules complexes sont revêtues de la membrane lipidique.

13. Composition selon l'une quelconque des revendications 10 à 12, dans laquelle le solvant organique polaire est un alcool.

14. Composition selon l'une quelconque des revendications 10 à 12, dans laquelle le solvant organique polaire est de l'éthanol.

15. Composition selon l'une quelconque des revendications 1 à 14, dans laquelle le lipide cationique est un ou plusieurs composés choisis parmi le chlorure de N-[1-(2,3-dioléoylpropyl)]-N,N,N-triméthylammonium, la N-[1-(2,3-dioléoylpropyl)]-N,N-diméthylamine, le chlorure de N-[1-(2,3-dioleyloxypropyl)-N,N,N-triméthylammonium, le bromure de N-[1-(2,3-ditétradécyloxypropyl)]-N,N-diméthyl-N-hydroxyéthylammonium et le 3β-[N-(N'N'-diméthylaminoéthyl) carbamoyl cholestérol.

16. Composition selon l'une quelconque des revendications 1 à 15, dans laquelle le dérivé de lipide, le dérivé d'acide gras ou le dérivé d'hydrocarbure aliphatique, d'une substance hydrosoluble est de la polyéthylène glycol phosphatidyl éthanolamine.

17. Composition selon l'une quelconque des revendications 1 à 16, dans laquelle le lipide neutre est de la phosphatidylcholine de jaune d'oeuf.
